# EUROPEAN PATENT SPECIFICATION

(11) **EP 1 546 354 B1**
(45) Date of publication and mention of the grant of the patent: **05.05.2010**
(21) Application number: 03791983.4
(22) Date of filing: 29.08.2003
(51) Int. Cl.: C12Q 1/68, C12P 19/34, C07H 21/02, C07H 21/04, C07H 19/00

(54) **ANALYTE DETECTION**
ANALYTNACHWEIS
DETECTION D'ANALYTES

(30) Priority: 29.08.2002 US 406893 P
(43) Date of publication of application: 29.06.2005
(73) Proprietor: GE Healthcare Bio-Sciences Corp., Piscataway, N.J. 08855-1327 (US)
(72) Inventor: SOOD, Anup, Flemington, NJ 08822 (US); KUMAR, Shiv, Belle Mead, NJ 08502 (US); FULLER, Carl, Berkeley Heights, NJ 07922 (US); NELSON, John, Clifton Park, NY 12065 (US)
(74) Representative: Franks, Barry Gerard
(86) International application number: PCT/US2003/027285
(87) International publication number: WO 2004/020603

(56) References cited:
- WO-A-03/020734
- WO-A2-03/020984
- US-A- 5 702 925
- US-A- 5 843 634
- US-A- 5 872 243
- US-A1- 2003 096 253
- US-A1- 2003 124 576
- US-B1- 6 403 339
- US-B1- 6 600 028
- VASSILIOU W ET AL: "Exploiting Polymerase Promiscuity: A Simple Colorimetric RNA Polymerase Assay" VIROLOGY, ACADEMIC PRESS,ORLANDO, US, vol. 274, no. 2, 1 September 2000 (2000-09-01), pages 429-437, XP004435943 ISSN: 0042-6822
- LUNDBERG K.S. ET AL.: 'High-fidelity amplification using a thermostable DNA polymerase isolated from pyrococcus furiosus' GENE vol. 108, no. 1, 1991, pages 1 - 6, XP002064945
- ASLANIDIS C. ET AL.: 'Ligation-independent cloning of PCR products (LIC-PCR)' NUCLEIC ACIDS RESEARCH vol. 18, no. 20, 20 September 1990, pages 6069 - 6074, XP000159869

## Description

### Field of Invention

The present invention relates generally to methods of detecting one or more analytes using terminal phosphate labeled nucleotides, including three or more phosphates as substrates for nucleic acid polymerases. The labels employed are enzyme-activatable and fluorescent.

### Background of Invention

Methods are known for detecting analytes in a sample with high specificity and sensitivity. These methods include antigen-antibody assays as well DNA hybridization based assays. Detection of analytes using immunodetection is well known in the art. Methods include direct labeling of antibodies using radioisotopes, fluorescent or chemiluminescent tags, or ELISA assays where an enzyme linked to the antibody catalyzes conversion of a chromogenic substrate to a detectable species. Latter are generally more desirable as multiple detectable moieties can be generated per binding event thereby increasing sensitivity. Similar methods have been incorporated into DNA hybridization based assays, which are generally more sensitive and in most diagnostic assays can be used at an earlier stage of disease progression. Enhanced sensitivity is achieved by first amplifying nucleic acid sequence based on the presence of a specific target sequence. Following amplification, the amplified sequences are detected and quantified. As a method of amplifying a nucleic acid sequence, the PCR (polymerase chain reaction) process is known. Presently, PCR is the most conventional means for *in vitro* amplification of nucleic acid. However, PCR has certain disadvantages, including the requirement for strict temperature control, inadequate quantification due to logarithmic amplification, and the danger of erroneous results brought about by simultaneous amplification of trace amounts of contaminated DNA. Additionally, except for nucleic acids, most other analytes are not easily amplified. In such cases, signal amplification methods are more desirable. These detect amplified decomposition products, i.e., a product or by-product of a reaction is amplified as the signal from a target analyte.

The traditional methods of amplifying the signal use enzyme linked to antibodies or polynucleotides and are limited by the amount of multiplexing one can achieve. There are only a few enzymes, such as alkaline phosphatase or horse radish peroxidase, that have been linked to antibodies or DNA probes. Other methods of signal amplification are based on nucleic acid metabolizing enzymes.

A cycling assay has been developed which utilizes λ-exonuclease to specifically cleave double stranded DNA (C.G. Copley et al., Bio Techniques, Vol. 13, No. 6, pp 882-892, 1992). This method involves hybridizing an oligonucleotide probe with a nucleic acid sequence complimentary thereto, allowing λ-exonuclease to act on the formed double-stranded DNA to decompose the hybridized probe. The probe is replaced by another probe, which is then decomposed. In this way, a cycling reaction repeats. In this method, the presence of a target DNA sequence is estimated by the detection of the decomposed probe. A disadvantage of this method is that the λ-exonuclease requires a probe which is phosphorylated at its 5'-terminal as the substrate. Following chemical synthesis of the probe by known methods, the 5'-terminal needs to be phosphorylated, and it is often difficult to confirm that all 5'-terminals are phosphorylated completely. An additional problem of this method is the low turnover number of cycling reactions, i.e., the number of times hybridization between the primer and target nucleotide occurs. The turnover number is low since the hybridization step must repeatedly occur.

An additional cycling assay by an exonuclease has been disclosed in EP 500224/A1: In this method, the synthesis of a DNA strand complimentary to a target DNA proceeds from a primer simultaneously with the decomposition of the same primer from the other side by a 5' → 3' exonuclease such that another primer hybridizes with the target sequence in place of the decomposed primer hybridized before. Therefore, in a single cycle reaction both the synthesis of a complimentary strand by DNA polymerase as well as the degradation of the synthesized strand repeatedly occurs. A disadvantage of this method is the low turnover number, with the hybridization step being rate limiting in that it must repeatedly occur.

A further cycling assay for detection of a polynucleotide containing a specific sequence is disclosed in U.S. Patent No. 5,849,487. This method relies on signal amplification and detection of decomposition products. This method includes using a combination of nucleic acid polymerase, 3'→ 5' exonuclease, a nuclease-resistant primer, a target nucleic acid, which may be DNA at limiting concentration, and at least one deoxynucleoside triphosphate (dNTP) to detect the target nucleic acid sequence. The method further includes synthesizing a complimentary strand being a nucleotide species located adjacent to the 3'-terminal of the nuclease-resistant primer, followed by decomposition of the nucleotide species joined to the end of the primer and detection of the resulting pyrophosphoric acid or deoxynucleoside monophosphate, the synthesis and decomposition of the nucleotide species being repeated one or more times. A disadvantage of this method as well as other detection methods presently widely in use is the need to separate labeled starting material from a final labeled product or by-product. Such separations generally require gel electrophoresis or immobilization of a target nucleic acid sequence onto a membrane for detection. For example, in U.S. Patent No. 5,849,487, the deoxynucleoside monophosphate formed by a nuclease reaction is separated by chromotography and optically measured. Alternatively, the pyrophosphoric acid which is formed upon incorporation of a complimentary base by DNA polymerase may be allowed to react with adenosine-5'-phosphosulfate and adenosine triphosphate sulfurase to form adenosine triphosphate, which is then detected using a luciferin-luciferase reaction; this presents the disadvantage of requiring additional reagents and incubation steps.

It has been known that DNA and RNA polymerases are able to recognize and utilize nucleosides with a modification at or in place of the gamma position of the triphosphate moiety. It is further known that the ability of various polymerases to recognize and utilize gamma-modified nucleoside triphosphates appears to vary depending on the moiety attached to the gamma phosphate.

A colorimetric assay for monitoring RNA synthesis from RNA polymerases in the presence of a gamma-phosphate modified nucleotide has been reported (Ref. Vassiliou W, Epp JB, Wang BB, Del Vecchio AM, Widlanski T, Kao CC. Exploiting polymerase promiscuity: A simple colorimetric RNA polymerase assay.Virology. 2000 Sep 1;274(2):429-37). In this report, RNA polymerase reactions were performed in the presence of a gamma-modified, alkaline phosphatase resistant nucleoside triphosphate which was modified at its gamma phosphate with a dinitrophenyl group. When RNA polymerase reactions were performed in the presence of this gamma-modified NTP as the sole nucleoside triphosphate and a homopolymeric template, it was found that RNA polymerase could recognize and utilize the modified NTP. Moreover, when the polymerase reactions were performed in the presence of an alkaline phosphatase, which digested the p-nitrophenyl pyrophosphate aldo-product of a phosphoryl transfer to the chromogenic p-nitrophenylate, an increase in absorbance was reported. A disadvantage of this detection method is that the real-time colorimetric assay, performed in the presence of an alkaline phosphatase, only works with a homopolymeric template.

It would, therefore, be of benefit to provide methods of detecting and characterizing an analyte, which methods would include utilization of terminal-phosphate-labeled nucleotides as substrates for DNA polymerase in a cycling assay by an exonuclease. It would further be of benefit if such methods would employ enzyme-activatable labels at the terminal phosphate of the nucleotide for production of an amplified detectable species from a target nucleic acid which would eliminate the need to separate labeled starting materials from labeled products or by-products. Moreover, it would be highly desirable if such methods for detecting and characterizing nucleic acids would allow for real-time monitoring of a heteropolymeric target nucleic acid using routine lab instrumentation. Finally, it is additionally desirable if such methods are easily multiplexed to analyze four or more analytes per reaction compartment simultaneously or in sequential manner.

### Summary of Invention

An aspect of the present invention is to provide a method of detecting an analyte comprising the steps of; (a) anchoring said analyte to a nucleic acid template; (b) conducting a nucleic acid polymerase reaction to produce labeled polyphosphate, said reaction comprising the reaction of said template, a primer, at least one terminal phosphate-labeled nucleotide, and a nucleic acid polymerase; and (c) analyzing said labeled polyphosphate by fluorescent detection.

A further aspect of the present invention is to provide a method of detecting an analyte in which a 3'→ 5' DNA exonuclease acting on DNA, is used together with DNA polymerase , a phosphatase and a primer-template combination with an anchoring moiety useful for anchoring the primer, the template or primer-template complex to the analyte so that a signal from the target analyte can be amplified and detected without the need for further operations such as separation of labeled reaction products from labeled starting materials.

The present invention provides methods for detecting an analyte. One method includes the steps of: (a) anchoring a nucleic acid template with an anchoring moiety to the target analyte, (b) conducting a DNA polymerase reaction, the reaction including the reaction of the template, a non-hydrolyzable primer, at least one terminal phosphate-labeled nucleotide, DNA polymerase and an enzyme having 3'→ 5' exonuclease activity, wherein the enzyme may be selected from DNA polymerases, exonucleases, and combinations thereof, which reaction results in the production of labeled polyphosphate; (c) permitting the labeled polyphosphate to react with a phosphatase to produce a detectable species; and (d) detecting the detectable species.

Another aspect of the invention relates to a method of detecting multiple analytes in a sample including the steps of: a) anchoring to each analyte a specific template nucleic acid with a unique base at the site opposite from the complementary nucleotide being added (b) conducting a DNA polymerase reaction, the reaction including the reaction of the template, a non-hydrolyzable primer, two or more terminal phosphate-labeled nucleotides with different labels, DNA polymerase and an enzyme having 3' → 5' exonuclease activity, wherein the enzyme may be selected from DNA polymerases, exonucleases, and combinations thereof, which reaction results in the production of labeled polyphosphate; (c) permitting the labeled polyphosphate to react with a phosphatase to produce a detectable species; and (d) detecting the detectable species. With this method it is possible to detect four analytes simultaneously using the natural bases. Further multiplexing can be achieved by using unnatural bases that have their own complementary partners and are not readily misincorporated opposite other natural or unnatural bases. Examples of these unnatural bases are cited in Lei Wang et. al. J. Am. Chem. Soc. 2000, 122, 5010-5011 and references therein.

Another aspect of the current invention relates to an alternative way to analyze multiple analytes in a reaction compartment is to (a) attach a specific template nucleic acid sequence to each analyte (b) anchor the analytes to the reaction compartment surface; (c) conduct a DNA polymerase reaction, the reaction comprising the reaction of a particular template sequence on an analyte, a complementary non-hydrolyzable primer, at least one terminal phosphate-labeled nucleotide, DNA polymerase and an enzyme having 3' → 5' exonuclease activity, wherein the enzyme may be selected from DNA polymerases, exonucleases, and combinations thereof, which reaction results in the production of labeled polyphosphate; (d) permitting the labeled polyphosphate to react with a phosphatase to produce a detectable species; and (e) detecting the detectable species; (f) washing all the unanchored components and (g) repeating the process with a different non-hydrolyzable primer complementary to a target sequence of a different analyte.

Further provided is a method of detecting an analyte including the steps of: (a) anchoring a nucleic acid template to the target analyte, (b) conducting a DNA polymerase reaction, the reaction comprising the reaction of the template, a non-hydrolyzable primer, at least one terminal phosphate-labeled nucleotide having 4 or more phosphate groups in the polyphosphate chain, DNA polymerase and an enzyme having 3' → 5' exonuclease activity, wherein the enzyme may be selected from DNA polymerases, exonucleases and combinations thereof, which reaction results in the production of labeled polyphosphate; and (c) detecting the labeled polyphosphate.

Another aspect of the invention relates to a method of detecting multiple analytes in a sample including the steps of: (a) anchoring to the target analyte a nucleic acid template with a unique base at the site opposite from the complementary nucleotide being added, (b) conducting a DNA polymerase reaction, the reaction comprising the reaction of the template, a non-hydrolyzable primer, two or more terminal phosphate-labeled nucleotides having 4 or more phosphate groups in the polyphosphate chain and each bearing a different label, DNA polymerase and an enzyme having 3' → 5' exonuclease activity, wherein the enzyme may be selected from DNA polymerases, exonucleases and combinations thereof, which reaction results in the production of labeled polyphosphate; (c) detecting the labeled polyphosphate.

Another aspect of the current invention relates to detecting multiple analytes in a reaction compartment is to (a) anchor a unique nucleic acid template to each target analyte, (b) anchor the analytes to the reaction compartment surface; (c) conduct a DNA polymerase reaction, the reaction comprising the reaction of a particular template sequence on an analyte, a complementary non-hydrolyzable primer, at least one terminal phosphate-labeled nucleotides having 4 or more phosphate groups in the polyphosphate chain, DNA polymerase and an enzyme having 3' → 5' exonuclease activity, wherein the enzyme may be selected from DNA polymerases, exonucleases and combinations thereof, which reaction results in the production of labeled polyphosphate; (d) detecting the labeled polyphosphate; (e) washing all the unanchored components and (f) repeating the process with a different non-hydrolyzable primer complementary to a target sequence of a different analyte.

Another aspect of the invention relates to a method of detecting an analyte including the steps of: (a) anchoring a nucleic acid template to the target analyte, (b) conducting a DNA polymerase reaction, the reaction comprising the reaction of the template, a non-hydrolyzable primer, at least one terminal phosphate-labeled nucleotide having 4 or more phosphate groups in the polyphosphate chain, DNA polymerase and an enzyme having 3' → 5' exonuclease activity, wherein the enzyme may be selected from DNA polymerases, exonucleases and combinations thereof, which reaction results in the production of labeled polyphosphate; (c) permitting the labeled polyphosphate to react with a phosphatase to produce a detectable species; and (d) detecting the detectable species.

Another aspect of the invention relates to a method of detecting multiple analytes in a sample including the steps of: (a) anchoring a unique nucleic acid template to each target analyte, (b) conducting a DNA polymerase reaction, the reaction comprising the reaction of a particular template nucleic acid sequence on an analyte, a non-hydrolyzable primer, two or more terminal phosphate-labeled nucleotides having 4 or more phosphate groups in the polyphosphate chain and each bearing a different label, DNA polymerase and an enzyme having 3' → 5' exonuclease activity, wherein the enzyme may be selected from DNA polymerases, exonucleases and combinations thereof, which reaction results in the production of labeled polyphosphate; (c) permitting the labeled polyphosphate to react with a phosphatase to produce a detectable species; and (d) detecting the detectable species.

Another aspect of current invention relates to detection of multiple analytes in a reaction compartment is to (a) anchor a unique nucleic acid template to each target analyte, (b) anchor the analytes to the reaction compartment surface; (c) conduct a DNA polymerase reaction, the reaction comprising the reaction of a particular template sequence on an analyte, a complementary non-hydrolyzable primer, at least one terminal phosphate-labeled nucleotides having 4 or more phosphate groups in the polyphosphate chain, a DNA polymerase and an enzyme having 3' → 5' exonuclease activity, wherein the enzyme may be selected from DNA polymerases, exonucleases and combinations thereof, which reaction results in the production of labeled polyphosphate; (d) permitting the labeled polyphosphate to react with a phosphatase to produce a detectable species; (e) detecting the detectable species; (f) washing all the unanchored components and repeating the process with a different non-hydrolyzable primer complementary to a target sequence of a different anal yte.

The invention further provides methods of characterizing an analyte. For example, the invention provides a method including the steps of: (a) anchoring a nucleic acid template to the target analyte, (b) conducting a DNA polymerase reaction, the reaction including the reaction of the template, a non-hydrolyzable primer, at least one terminal phosphate-labeled nucleotide, DNA polymerase and an enzyme having 3' → 5' exonuclease activity, wherein the enzyme may be a DNA polymerase, exonuclease or a combination thereof, which reaction results in the production of labeled polyphosphate; (b) permitting the labeled polyphosphate to react with a phosphatase to produce a detectable species; (c) detecting the detectable species; and (d) characterizing the analyte based on the detection.

Further encompassed by the invention is a method of characterizing an analyte including the steps of: (a) anchoring a nucleic acid template to the target analyte, (b) conducting a DNA polymerase reaction, the reaction comprising the reaction of the template, a non-hydrolyzable primer, at least one terminal phosphate-labeled nucleotide having 4 or more phosphate groups in the polyphosphate chain, DNA polymerase and an enzyme having 3' → 5' exonuclease activity, wherein the enzyme may be selected from DNA polymerases, exonucleases and combinations thereof, which reaction results in the production of labeled polyphosphate; (c) detecting said labeled polyphosphate; and (d) characterizing the analyte based on the detection.

Also provided is a method of detecting an analyte including the steps of:
(a) anchoring a nucleic acid template to the target analyte, (b) conducting a DNA polymerase reaction, the reaction comprising the reaction of the template, a non-hydrolyzable primer, at least one terminal phosphate-labeled nucleotide having 4 or more phosphate groups in the polyphosphate chain, DNA polymerase and an enzyme having 3' → 5' exonuclease activity, wherein the enzyme may be selected from DNA polymerases, exonucleases and combinations thereof, which reaction results in the production of labeled polyphosphate; (c) permitting the labeled polyphosphate to react with an alkaline phosphatase to produce a detectable species having a signal profile characteristic of the analyte; (d) detecting the detectable species; and (e) characterizing the analyte based on the signal profile.

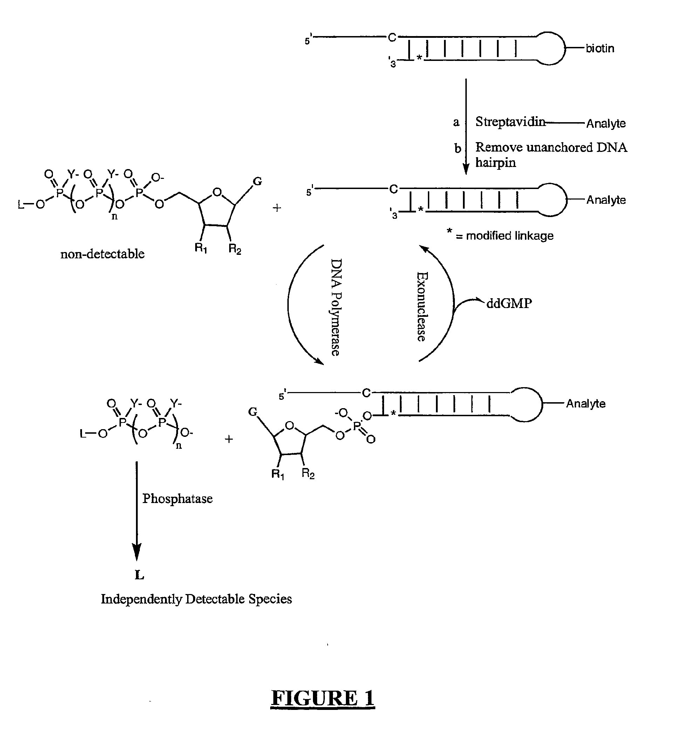

Similar methods as described above can be used for characterization of multiple analytes in a reaction compartment. In the specifications listed above it is equally feasible to attach the non-hydrolyzable primer to the analyte and provide the template in solution. In some preferred embodiments of the above specifications, the non-hydrolyzable primer and template are joined together in a hair-pin structure shown in Figure 1 and the analyte binding functionality is on the loop of the hairpin oligonucleotide. In most preferred embodiments of above specifications, before conducting the DNA polymerase reaction, unanchored nucleic acid template is removed from the anchored material. Depending on the target analyte this can be achieved by a number of methods including simple washing, precipitation, filteration, or by chromatographic or electrophoretic methods.

Further encompassed by the invention are kits for detecting an analyte, one kit including: (a) at least one terminal-phosphate-labeled nucleotide; (b) a DNA polymerase; (c) a phosphatase, (d) a template and a complementary non-hydrolyzable primer, one of which bears an anchoring moiety; and (e) a nuclease with enzymatic activity sufficient to decompose DNA in the 3' → 5' direction.

A further kit for detection of an analyte is provided which includes: (a) at least one terminal-phosphate-labeled nucleotide; (b) a phosphatase, (c) a template and a complementary non-hydrolyzable primer, one of which bears an anchoring moiety; and (d) a DNA polymerase with enzymatic activity sufficient to decompose DNA in the 3' → 5' direction.

A further aspect of the present invention is to provide a kit for the detection of an analyte, one kit including: (a) at least one terminal-phosphate-labeled nucleotide; (b) a DNA polymerase; (c) a phosphatase; (d) a hairpin template-primer combination with an anchoring moiety and a non-hydrolyzable 3'-end; and (e) a nuclease with enzymatic activity sufficient to decompose DNA in the 3' → 5' direction.

Lastly, a kit is provided herein for the detection of an analyte which includes: (a) at least one terminal-phosphate-labeled nucleotide; (b) a phosphatase (c) a hairpin template-primer combination with an anchoring moiety and a non-hydrolyzable 3'-end; and (d) a DNA polymerase with enzymatic activity sufficient to decompose DNA in a 3' → 5' direction.

### Brief Description of Drawings

Figure 1 shows an embodiment of a method of the present invention where a hairpin looped DNA primer-template with non-hydrolyzable 3'-end is attached to the target analyte and a terminal-phosphate-labeled nucleotide complimentary in sequence to template is joined to the 3' end of a nuclease-resistant primer, followed by decomposition thereof to effect a cycling assay in which the labeled polyphosphate by-product of nucleotide incorporation reacts with an alkaline phosphatase to produce a detectable species.

Figure 2 is a graph of time versus fluorescence emission obtained by the use of a 5' → 3' exonuclease to amplify signal generated by incorporation of nucleotides labeled on the terminal phosphate with fluorogenic dyes.

Figure 3 (A and B) shows bar graphs of the fluorescence emission obtained by the use of a 5' → 3' exonuclease to amplify signal generated by the sequence specific incorporation of nucleotides labeled on the terminal phosphate with fluorogenic dyes.

### Description of Invention

The term analyte as defined herein includes, but is not limited to, a biomolecule, a whole cell or a commercially important substrate that may need to be tracked for its distribution or identification. Biomolecules include nucleic acids, peptides, proteins, oligosaccharides, lipids, antigens, etc., while commercially important substrates include, but are not limited to, organic and inorganic polymers or products made therefrom.

The term "phosphatase" as defined herein includes alkaline and acid phosphatases, 5'-nucleotidases, and phosphate or polyphosphate transferring enzymes which can not cleave a terminal phosphate labeled nucleoside polyphosphate, but after incorporation of the nucleoside monophosphate by a polymerase, can remove the phosphate units from the resultant dye polyphosphate.

The term "nucleoside" as defined herein is a compound including a purine deazapurine, or pyrimidine base linked to a sugar or a sugar substitute, such as a carbocyclic or acyclic linker at the 1' position or equivalent position and includes 2'-deoxy and 2'-hydroxyl, 2', 3'-dideoxy forms, as well as other substitutions.

The term "nucleotide" as used herein refers to a phosphate ester of a nucleoside, wherein the esterification site typically corresponds to the hydroxyl group attached to the C-5 position of the pentose sugar.

The term "oligonucleotide" includes linear oligomers of nucleotides or derivatives thereof, including deoxyribonucleosides, ribonucleosides, and the like. Throughout the specification, whenever an oligonucleotide is represented by a sequence of letters, the nucleotides are in the 5' → 3' order from left to right where A denotes deoxyadenosine, C denotes deoxycytidine, G denotes deoxyguanosine, and T denotes thymidine, unless noted otherwise.

The term "primer" refers to a linear oligonucleotide that anneals in a specific way to a unique nucleic acid sequence and allows for amplification of that unique sequence.

The phrase "target nucleic acid sequence" and the like refers to a nucleic acid sequence to which the primer is targeted.

The term "anchoring" means attaching through covalent or non-covalent interactions.

The present invention relates to methods of detecting and characterizing one or more analytes in a sample wherein the analyte is marked by the attachment of a nucleic acid template or a non-hydrolyzable primer or a combination thereof in the form of a DNA hairpin and a convenient assay is used for monitoring the addition of a terminal-phosphate-labeled nucleotide which is complimentary to a specific base in the target nucleic acid, onto the 3'-terminal of a non-hydrolyzable primer followed by nuclease decomposition thereof. DNA polymerases synthesize oligonucleotides via transfer of a nucleoside monophosphate from a deoxynucleoside triphosphate (dNTP) to the 3' hydroxyl of a growing oligonucleotide chain.

The force which drives this reaction is the cleavage of an anhydride bond and the con-commitant formation of an inorganic pyrophosphate. The present invention utilizes the finding that structural modification of the terminal-phosphate of the nucleotide does not abolish its ability to function in the polymerase reaction. The oligonucleotide synthesis reaction involves direct changes only at the α- and β-phosphoryl groups of the nucleotide, allowing nucleotides with modifications at the terminal phosphate position to be valuable as substrates for nucleic acid polymerase reactions.

The methods provided by this invention utilize a nucleoside polyphosphate analogue, such as a deoxynucleoside polyphosphate or dideoxynucleoside polyphosphate analogue with an electrochemical label, mass tag, or a chromogenic, chemiluminescent, or fluorescent dye label attached to the terminal-phosphate. When a nucleic acid polymerase uses this analogue as a substrate, an enzyme-activatable label is present on the inorganic polyphosphate by-product of phosphoryl transfer. Cleavage of the polyphosphate product of phosphoryl transfer by a phosphatase, results in a detectable change in the label attached thereon. For example, if 3-cyanoumbelliferone dye is attached via its hydroxyl group to the terminal phosphate position of a nucleotide, the dye is not fluorescent when excited at 408 nm and it is not a substrate for alkaline phosphatase. Once this nucleotide is incorporated into DNA, the released dye inorganic polyphosphate (which also is not fluorescent when excited at 408 nm) is a substrate for alkaline phosphatase. Once de-phosphorylated, the dye becomes fluorescent when excited at 408 nm and hence detectable. The specific analysis of the polyphosphate product can be carried out in the same reaction solution as, the polymerase and exonuclease reactions, with no need to separate reaction products from starting materials. This allows for the detection and, optionally, quantification of nucleic acids formed during polymerase reactions and hence an analyte if the nucleic acid is attached to the analyte using routine instrumentation such as fluorimeters or spectrophotometers.

It is noted that while RNA and DNA polymerases are able to recognize nucleotides with modified terminal phosphoryl groups, the inventors have determined that this starting material is not a substrate for phosphatases. The scheme below shows relevant molecules in the method of this invention; namely the terminal-phosphate-labeled nucleotide, the labeled polyphosphate by-product and the enzyme-activated label.

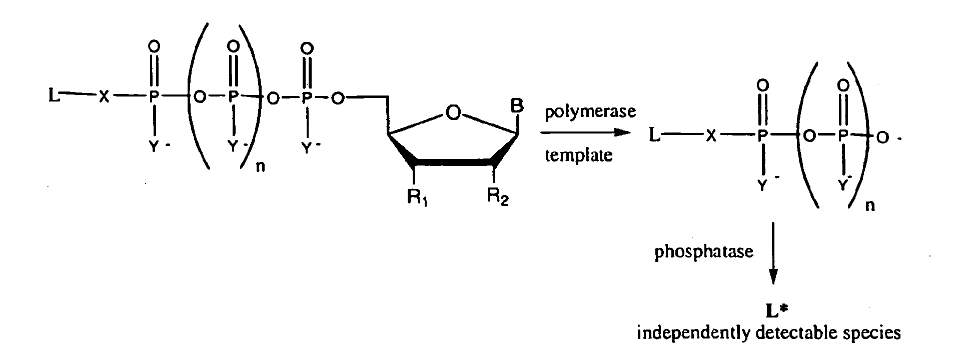

In the scheme above, n is 1 or greater, R1 and R2 are independently H, SH, SR, F, Br, Cl, I, N3, NH2, NHR, OR or OH; B is a natural or modified nucleoside base; X is O, S, or NH; Y is O, S, or BH3 and L is a phosphatase activatable label which may be a chromogenic, fluorogenic, or chemiluminescent molecule, mass tag or electrochemically detectable moiety. A mass tag is a small molecular weight moiety suitable for mass spectrometry that is readily distinguishable from other reaction products due to difference in mass. An electrochemical tag is an easily oxidizable or reducible species. It has been discovered that when n is 2 or greater, the nucleotides are significantly better substrates for polymerases than when n is 1. Therefore, in preferred embodiments of the present invention, n is 2, 3 or 4. In further desired embodiments of the present invention, X and Y are O; and R1 and R2 are independently H or OH; B is a nucleoside base and L is a label which may be a chromogenic, fluorogenic or a chemiluminescent molecule.

In one embodiment of the method of detecting an analyte provided herein, the steps include anchoring a nucleic acid template to the analyte, conducting a DNA polymerase reaction, the reaction including the reaction of the template, a non-hydrolyzable primer, at least one terminal phosphate-labeled nucleotide, DNA polymerase and an enzyme having 3' → 5' exonuclease activity, wherein the enzyme may be selected from DNA polymerases, exonucleases and combinations thereof, which reaction results in the production of labeled polyphosphate provided the terminal phosphate-labeled nucleotide is complementary to the template; permitting the labeled polyphosphate to react with a phosphatase, such as alkaline phosphatase, to produce a detectable species; and detecting the detectable species.

In the methods of characterizing analyte sample provided by this invention, the target analyte may be characterized by determining the presence or absence of the detectable species. Moreover, the detectable species may have a characteristic staining profile or signal profile associated with it, the profile being characteristic of the sample. This allows for characterization of the analyte target based on the unique profile of the detectable species.

Figure 1 shows the general scheme employed for each of the methods described above. In this scheme, n is 1 or greater, R1 and R2 are independently H, OH, SH, SR, F, Cl, Br, I, N3, NH2 or OR; G is guanine, or representative of a natural or modified nucleoside base; C is cytosine or representative of the base complimentary to the added nucleotide; Y is O, S, or BH3 and L is a chromogenic, fluorogenic, chemiluminescent, or electrochemical label or mass tag which preferably becomes independently detectable when the phosphate is removed. As shown in Figure 1, a DNA hairpin with non-hydrolyzable, but extendable 3'-end is attached to an analyte. After separation of the unbound DNA hairpin, the DNA polymerase reaction is conducted in the presence of the hairpin anchored analyte and at least one terminal-phosphate-labeled nucleotide under conditions to cause a nucleoside monophosphate derived from the terminal-phosphate-labeled nucleotide to join to the 3'-terminal end of the nuclease-resistant DNA hairpin. This is accompanied by the concomitant formation of a labeled product which may not be independently detectable. The labeled polyphosphate concomitantly formed during incorporation of the nucleotide species is permitted to react with a phosphatase to produce an independently detectable species which serves as the signal from the target polynucleotide. Addition of a complimentary nucleotide species to the 3'-terminal of the primer is followed by decomposition thereof by the reaction of a 3' → 5' exonuclease which may be associated with the DNA polymerase itself. The synthesis and decomposition of the complementary strand being essentially the nucleotide species, is repeated one or more times to effect a cycling assay.

In the methods described above, the polymerase reaction may be conducted in the presence of a phosphatase, such as alkaline phosphatase or a phosphate transferring enzyme, which converts the labeled polyphosphate product to the detectable label. As such, convenient assays are established for detecting and characterizing an analyte that allows for continuous, real-time monitoring of detectable species formation. This represents a homogeneous assay format in that it can be performed in a single tube using analytes pre-anchored with DNA template, primer or combined template primer hairpin.

It is noted that in embodiments including terminal phosphate-labeled nucleotides having four or more phosphates in the polyphosphate chain, it is within the contemplation of the present invention that the labeled polyphosphate by-product of phosphoryl transfer may be detected without the use of phosphatase treatment. For example, it is known that natural or modified nucleoside bases, particularly guanine, can cause quenching of fluorescent markers. Therefore, in a terminal phosphate labeled nucleotide, the label may be partially quenched by the base. Upon incorporation of the nucleoside monophosphate, the labeled polyphosphate by-product may be detected due to its enhanced fluorescence. Alternatively, it is possible to physically separate the labeled polyphosphate product by chromatographic separation methods before identification by fluorescence, color, chemiluminescence, or electrochemical detection. In addition, mass spectrometry could be used to detect the products by mass difference.

The detectable species may be produced in amounts substantially proportional to the amount of target analyte and, as such, is a signal for the amount of the target analyte. The methods herein described may further include the step of quantifying the target analyte based on the amount of detectable species produced during the reaction. The step of quantifying the target analyte is desired to be done by comparison of spectra produced by the detectable species with known target quantities.

In the present invention, once hybridized, the oligonucleotide primer can repeatedly function so as to permit the reaction to proceed quantitatively in an at least equal molar amount relative to the template nucleotide sequence. When used separately the ratio of the oligonucleotide template to primer useful in the methods of the present invention should be that sufficient to attain a favorable hybridization. In general, a sensitive assay can be attained by the presence of a template primer ratio of one and desirably in a 5-fold excess of one over the other.

The methods provided by the present invention may further include the step of including one or more additional detection agents in the DNA polymerase reaction. The additional detection agent may be capable of a response which is detectably different from the detectable species. For example, the additional detection agent may be an antibody.

The target analyte of the present invention includes, but is not limited to, biomolecules such as nucleic acids, peptides, proteins, antigens, lipids, complex sugars; whole cells and synthetic polymers and/or substrates.

The terminal-phosphate-labeled nucleotide useful in the methods and kits of the present invention may be represented by Formula I:

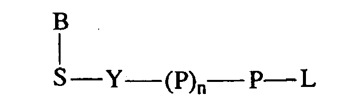

wherein P=phosphate (PO₃) and derivatives thereof, n is 2 or greater; Y is an oxygen or sulfur atom; B is a nitrogen-containing heterocyclic base; S is an acyclic moiety, carbocyclic moiety or sugar moiety; L is an enzyme-activatable label containing a hydroxyl group, a sulfhydryl group or an amino group suitable for forming a phosphate ester, a thioester or a phosphoramidate linkage at the terminal phosphate of a natural or modified nucleotide; P-L is a phosphorylated label which preferably becomes independently detectable when the phosphate is removed.

For purposes of the methods of the present invention, useful carbocyclic moieties have been described by Ferraro, M. and Gotor, V. in Chem Rev. 2000, volume 100, 4319-48. Suitable sugar moieties are described by Joeng, L.S. et al., in J Med. Chem. 1993, vol. 356, 2627-38; by Kim H.O. et al., in J Med. Chem. 193, vol. 36, 30-7; and by Eschenmosser A., in Science 1999, vol. 284, 2118-2124. Moreover, useful acyclic moieties have been described by Martinez, C.I., et al., in Nucleic Acids Research 1999, vol. 27, 1271-1274; by Martinez, C.I., et al., in Bioorganic & Medicinal Chemistry Letters 1997, vol. 7, 3013-3016; and in U.S. Patent 5,558,991 to Trainer, G.L. Structures for these moieties are shown below, where for all moieties R may be H, OH, NHR, lower alkyl and aryl; for the sugar moieties X and Y are independently O, S, or NH; and for the acyclic moieties, X = O, S, NH, NR.

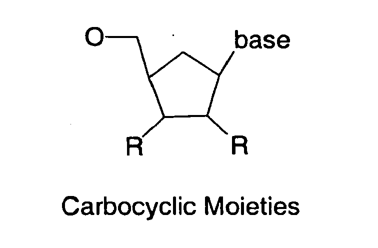

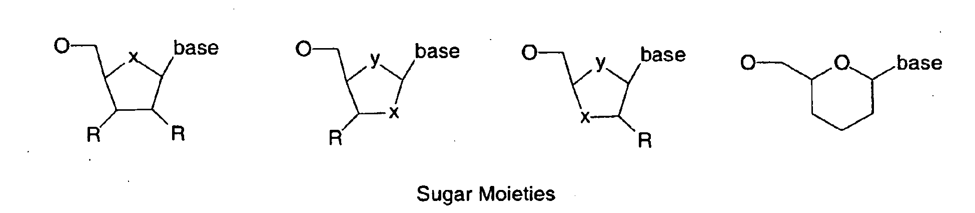

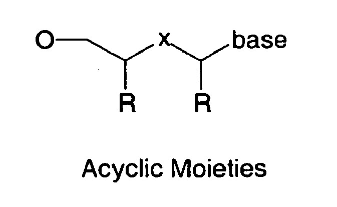

In certain embodiments, the sugar moiety may be selected from the following: ribosyl, 2'-deoxyribosyl, 3'-deoxyribosyl, 2',3'-dideoxyribosyl, 2', 3'-didehydrodideoxyribosyl, 2'-alkoxyribosyl, 2'-azidoribosyl, 2'-aminoribosyl, 2'-fluororibosyl, 2'mercaptoriboxyl, 2'-alkylthioribosyl, carbocyclic, acyclic and other modified sugars.

Moreover, in Formula I above, the base may include uracil, thymine, cytosine, 5-methylcytosine, guanine, 7-deazaguanine, hypoxanthine, 7-deazahypoxanthine, adenine, 7- deazaadenine, 2,6-diaminopurine or analogs thereof.

The enzyme-activatable label attached at the terminal phosphate position of the nucleotide may be selected from 1,2-dioxetane chemiluminescent compounds, fluorogenic dyes, chromogenic dyes, mass tags, electrochemical tags or combinations thereof. This would allow the detectable species to be detectable by the presence of any one of color, fluorescence emission, chemiluminescence, or a combination thereof.

The enzyme-activatable label may also be a chemical moiety that becomes a substrate for an additional chemical or enzymatic reaction that results in the production of a detectable signal.

Wherein the phosphorylated label shown in Formula I above is a fluorogenic moiety, it is desirably selected from one of the following examples (shown as their phosphate esters): 2-(5'-chloro-2'-phosphoryloxyphenyl)-6-chloro-4-(3H)-quinazolinone, sold under the trade name ELF 97 (Molecular Probes, Inc.), fluorescein diphosphate, fluorescein 3'(6')-O-alkyl-6'(3')-phosphate, 9H-(1,3-dichloro-9,9-dimethylacridin-2-one-7-yl)phosphate, 4-methylumbelliferyl phosphate, resorufin phosphate, 4-trifluoromethylumbelliferyl phosphate, umbelliferyl phosphate, 3-cyanoumbelliferyl phosphate, 9,9-dimethylacridin-2-one-7-yl phosphate, and 6,8-difluoro-4-methylumbelliferyl phosphate. Structures of these dyes are shown below:

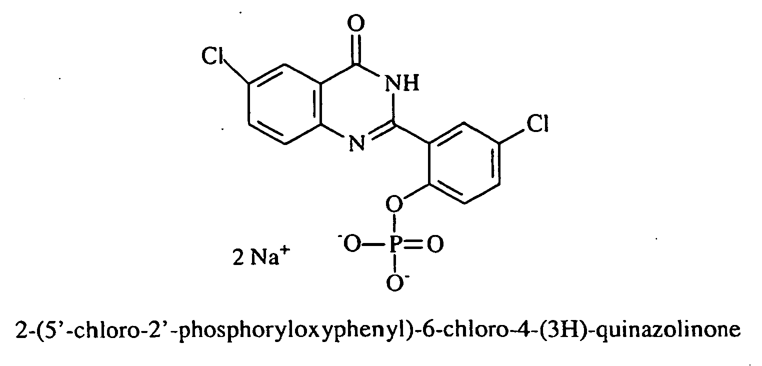

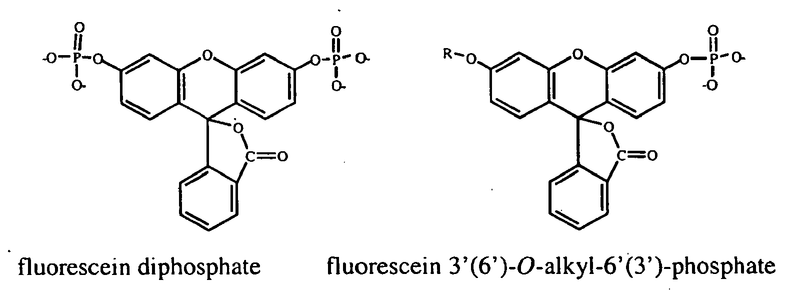

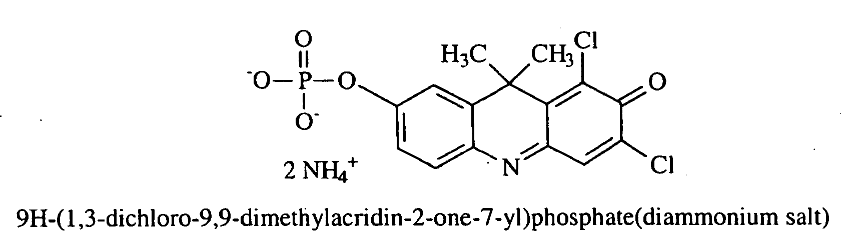

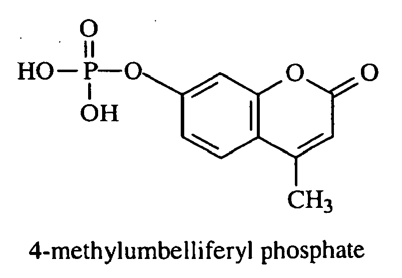

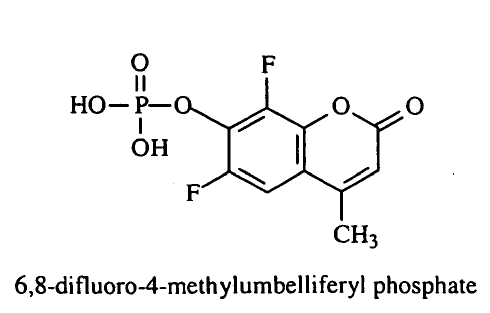

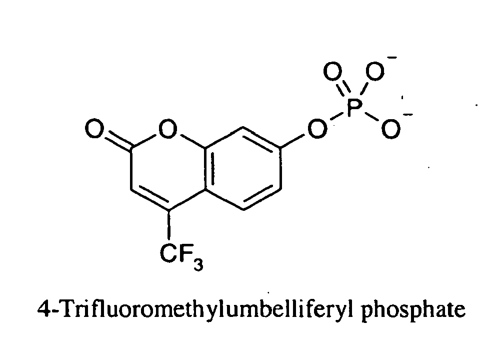

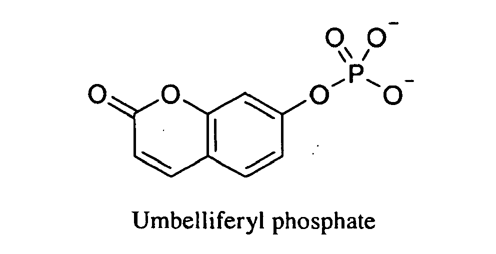

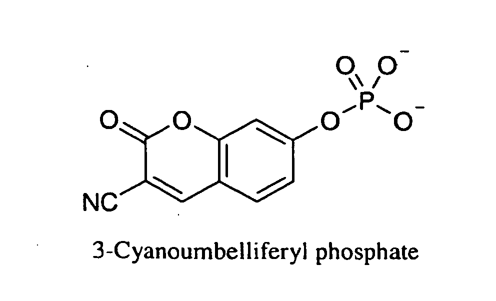

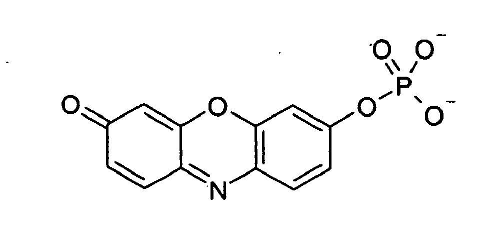

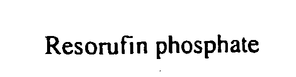

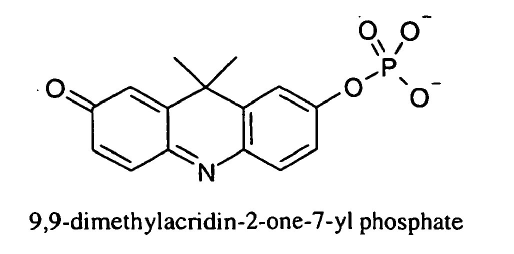

Wherein the phosphorylated label shown in Formula I above is a chromogenic moiety, it may be selected from the following moieties (shown as the phosphate esters): 5-bromo-4-chloro-3-indolyl phosphate, 3-indolyl phosphate, p-nitrophenyl phosphate and derivatives thereof. The structures of these chromogenic dyes are shown below:

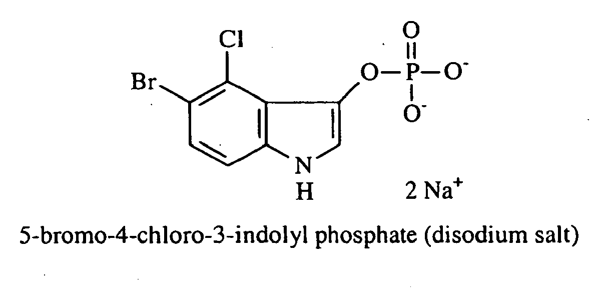

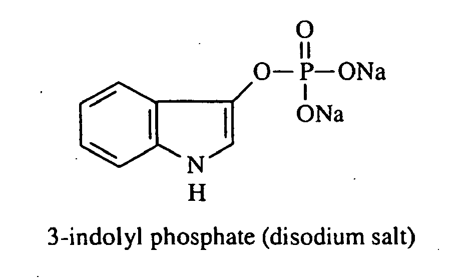

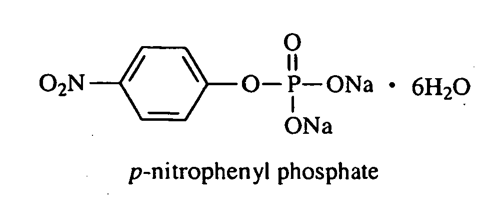

The moiety at the terminal phosphate position may further be a chemiluminescent compound wherein it is desired that it is an alkaline phosphatase-activated 1,2-dioxetane compound. The phosphate esters of the 1,2-dioxetane compound may include, but are not limited to, disodium 2-chloro-5-(4-methoxyspiro[1,2-dioxetane-3,2'-(5-chloro-)tricyclo[3,3,1-1^{3.7}]-decan]-1-yl)-1-phenyl phosphate, sold under the trade name CDP-*Star* (Tropix, Inc., Bedford, MA), chloroadamant-2'-ylidenemethoxyphenoxy phosphorylated dioxetane, sold under the trade name CSPD (Tropix), and 3-(2'-spiroadamantane)-4-methoxy-4-(3"-phosphoryloxy)phenyl-1,2-dioxetane, sold under the trade name AMPPD (Tropix). The structures of these commercially available dioxetane compounds are disclosed in US patents 5,582,980, 5,112,960 and 4,978,614, respectively, and are shown below:

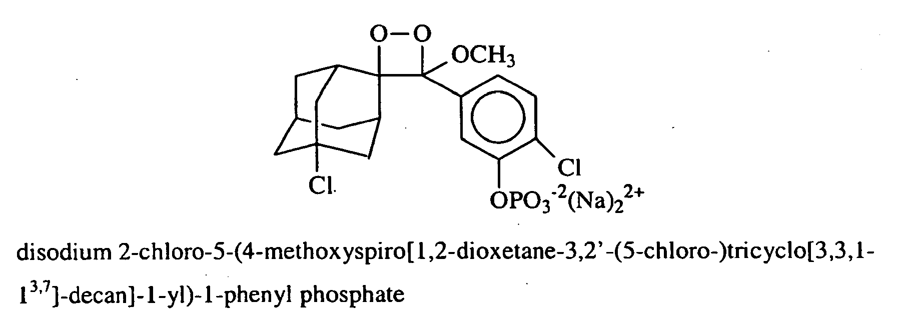

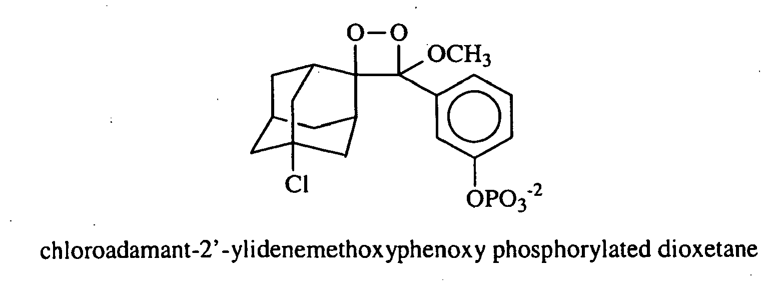

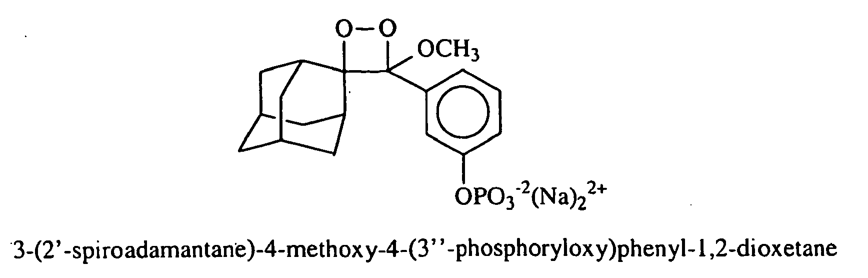

In the methods of the present invention, the non-hydrolyzable primer should be nuclease-resistant in order to prevent its decomposition by the 3' → 5' exonuclease present in the system.

As described above, the 3' → 5' exonuclease activity may be associated with the DNA polymerase itself. Suitable DNA polymerases for use in the present invention include, but are not limited to, the Klenow fragment of DNA polymerase I, Phi 29 DNA polymerase, DNA polymerase I, T4 DNA polymerase Thermo Sequenase (Amersham Biosciences Corporation), Amplitaq FS (Applied Biosystems), reverse transcriptase, and T7 DNA polymerase.

Methods for synthesizing nuclease-resistant oligonucleotide primers are not particularly limited, and any suitable method known in the art may be used. For example, in one embodiment of the method provided by the invention, the non-hydrolyzable primer is phosphorothioated at the 3'-most phosphodiester linkage terminal. Methods of chemically synthesizing an oligonucleotide primer having nuclease resistance by introducing a phosphorothioate bond into the target site of the primer are well known. In one method, the primer may be chemically synthesized using a modified phosphoramidite method in which the usual oxidation step by iodine water is replaced with an oxidation treatment with a reagent suitable for phosphorothioation, such that a phosphorothioate bond may be introduced in place of the usual phosphodiester bond. One suitable reagent for phosphorothioation is Beaucage's Reagent (3H-1,2-benzodithiole-3-one 1,1-dioxide). This method can be used to introduce a phosphorothioate bond into the primer at any chosen site, including at the 3'- most phosphodiester linkage.

An alternative means of preparing an oligonucleotide primer with a phosphorothioate bond prior to the time of analysis, is via DNA polymerase incorporation of a nucleotide analog in which an oxygen atom at the α-position is replaced by sulfur. Such substituted compounds are referred to as α-S-deoxynucleoside triphosphates. A DNA polymerase can incorporate the sulfur-substituted analog in place of deoxynucleoside triphosphate to give a phosphorothioated oligonucleotide primer containing nuclease resistance.

In any event, the presence of a phosphorothioate bond in place of a phosphodiester bond in the vicinity of the 3'-terminal of the oligonucleotide primer confers a resistance on the part of the primer to an exonuclease cleaving from the 3'-terminal side. The oligonucleotide primer is sufficiently non-hydrolyzable by the introduction of only a single phosphorothioate bond,

Methods for anchoring oligonucleotides to other substrates are well known in the art and include non-covalent binding such as biotin-streptavidin binding and covalent binding achieved by reacting functionalized oligonucleotides, e.g., amine functionalized oligonucleotides with activated acids, aldehydes, epoxides, etc., or thiol modified nucleotides with activated haloacetamide or vice versa.

Reaction conditions such as buffer, pH, and temperature should be selected to achieve sufficient hybridization, polymerase, nuclease, and phosphatase activities. Temperatures suitable for hybridization depend on the homology between the oligonucleotide primer and the target sequence, but are expected to be in the range of about 20° to about 60°C. The pH values are desired to be in the range of about 7 to 9 in a suitable buffer such as Tris-HCl buffer or HEPES.

The present invention is characterized in that following the anchoring step of a nucleic acid template or primer or a combination of two as described above to the analyte, at least one terminal-phosphate-labeled deoxynucleoside polyphosphate, a complementary oligonucleotide if not already present on the anchored analyte, a DNA polymerase, a nuclease (which may be associated with the polymerase), and a phosphatase are added to the system so that a nucleotide located next to the 3'-terminal of the primer and complimentary to the target nucleic acid is incorporated, followed by decomposition thereof and detection of a detectable species which acts as the signal from the target analyte, the synthesis and decomposition of the complimentary strand being repeated one or more times to effect a cycling assay for amplification of the signal.

It is well within the contemplation of the present invention that the amplification reaction could be performed using a polymerase and a single stranded nuclease (which could be an intrinsic property of the polymerase or a separate enzyme). The reaction is thermally cycled, allowing the extension of the primer by polymerase during low temperature, and removal of the added base by nuclease during high temperature. This would allow the user to control the amount of amplification, as it would be dependant on the number of thermal cycles which were performed.

### EXAMPLES

The following examples illustrate certain preferred embodiments of the illustration that are not intended to be illustrative of all embodiments.

### Example 1

### Preparation of γ (7-Hydroxy-3H-Phenoxazin-3-one)ddGTP (γ-Resorufin-ddGTP)

ddGTP (125 µl of 86.7 mM solution, 10.8 µmol) was coevaporated with anhydrous DMF (3x 0.25 ml). To this, DCC (5 eq.) was added and the mixture was again coevaporated with anhydrous DMF (0.25 ml). Residue was taken in anhydrous DMF (1 ml) and the reaction was stirred at room temperature over a weekend. Resorufin (20 eq.) was coevaporated with anhydrous DMF (2x 1 ml) and ddGTP trimetaphosphate from the above cyclization step was added, followed by 20 eq. of triethylamine. After 2 weeks, the reaction mixture was concentrated on a rotary evaporator and the residue was extracted with water (3x 2 ml) and filtered. The filtrate was purified on an Xterra RP C18 (19x100 mm) column using 0-30% acetonitrile in 0.1 M triethylammonium bicarbonate (pH 6.7) in 5 column volumes and 30-50% acetonitrile in 1 column volume. The pure fraction was concentrated on a rotavap and coevaporated with methanol (2 x 5 ml). The residue was dissolved in water (1.5 ml) to give a 0.5 mM solution. HPLC purity at 260 nm > 98%, at 470 nm > 97.5%. UV/VIS = 251 and 472 nm. MS: M-1 = 685.10 (calc. 685.03).

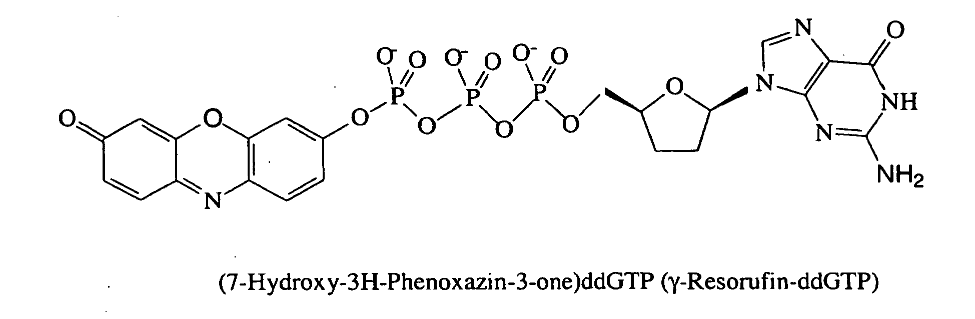

### Example 2

### Preparation of γ- (3-Cyanocoumarinyl)ddATP (γCNCoumarin-ddATP)

ddATP (100 µl of 89 mM solution, >96%) was coevaporated with anhydrous DMF (2x 1 ml). To this DCC (9.2 mg, 5 eq.) was added and mixture was again coevaporated with anhydrous DMF (1 ml). Residue was taken in anhydrous DMF (0.5 ml) and reaction was stirred at room temperature. After overnight 7-hydroxy-3-cyanocoumarin (33.3 mg, 20 eq.) and TEA (25 µl, 20 eq.), were added and mixture was stirred at RT. After 1 day, HPLC analysis indicated a major product (55% at 254 nm) at 8.1 min with another minor product at 10 min (~10%). No significant change occurred after another day. Reaction mixture was concentrated on rotary evaporator and residue was extracted with 3x2 ml water and filtered. Aq solution was concentrated and purified on C18 using 0-30% acetonitrile in 0.1M TEAB (pH 6.7) in 30 min and 30-50% acetonitrile in 10 min, flow rate 15 ml/min. Main peak was collected in 3 fractions. HPLC of the main peak (fr. 2) showed a purity of 95.6% at 254 nm and 98.1% at 335 nm. It was concentrated on rotary evaporator (at RT), coevaporated with MeOH (2x) and water (1x). Residue was dissolved in 0.5 ml water. A 5 µl sample was diluted to 1 ml for UV analysis. A346 nm = 0.784. Assuming an extinction coeff. of 20,000 (reported for 7-ethoxy-3-cyanocoumarin, Molecular Probes Catalog), concentration = 7.84 mM. Yield = 3.92 µmol, 44%. Sample was repurified on C18 column using same method as above. Sample peak was collected in 3 fractions. Fractions 2 & 3, with >98% purity at 254 nm and >99.5% purity at 340 nm, were combined. After concentration, residue was coevaporated with MeOH (2x) and water (1x). Sample was dissolved in water (1 ml) to give a 2.77 mM solution. MS: M- = 642.98 au (calc 643.00 au), UV λ_{A} = 263 & 346 nm. The cyanocoumarin dye attached to the gamma phosphate of ddATP is fluorescent with an excitation maximum of 346 nm and an emission maximum of about 411 nm. Upon hydrolysis of the phosphate ester to release the free coumarin dye, the spectrum changes with excitation maximum of about 408 nm and emission maximum of about 450 nm. This change is readily detected by simple fluorescence measurements or color change. Synthesis of gamma nucleotides has been generally described by Arzumanov, A, et al in J Biol Chem. (1996) Oct 4;271(40):24389-94.

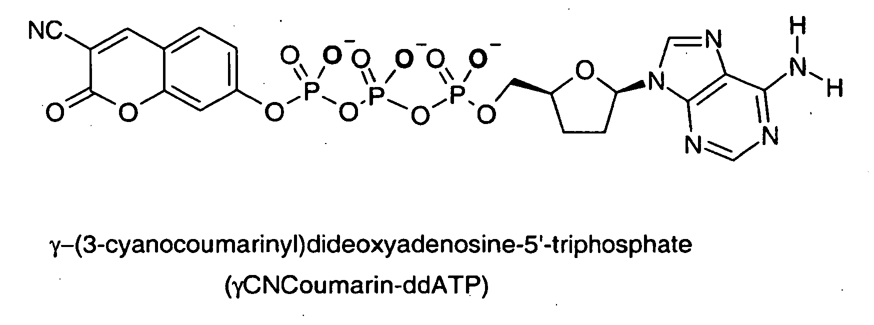

### Example 3

### Preparation of δ-9H(1,3-dichloro-9,9-dimethylacridin-2-one-7-yl)-dideoxythymidine-5'-tetraphosphate (ddT4P-DDAO)

ddTTP (100 µl of 80 mM solution) was coevaporated with anhydrous dimethylformamide (DMF, 2x 1 ml). To this dicyclohexylcarbodimide (8.3 mg. 5 eq.) was added and the mixture was again coevaporated with anhydrous DMF (1 ml). Residue was taken in anhydrous DMF (1 ml) and reaction was stirred at room temperature overnight. HPLC showed mostly cyclized triphosphate (~82%). Reaction mixture was concentrated and residue was washed with anhydrous diethyl ether 3x. It was redissolved in anhydrous DMF and concentrated to dryness on rotavap. Residue was taken with DDAO-monophosphate, ammonium salt (5 mg, 1.5 eq.) in 200 µl anhydrous DMF and stirred at 40°C over the weekend. HPLC showed formation of a new product with desired UV characteristics at 11.96 min. (HPLC Method: 0.30% acetonitrile in 0.1M triethylammonium acetate (pH 7) in 15 min, and 30-50% acetonitrile in 5 min, Novapak C-18 3.9x150 mm column, 1 ml/min). LCMS (ES-) also showed a major mass peak 834 for M-1 peak. Reaction mixture was concentrated and purified on Deltapak C18, 19x 300 mm column using 0.1M TEAB (pH 6.7) and acetonitrile. Fraction with product was repurified by HPLC using the same method as described above. Fraction with pure product was concentrated, coevaporated with MeOH (2x) and water (1x). Residue was dissolved in water (1.2 ml) to give a 1.23 mM solution. HPCL purity as 254 nm > 97.5%, at 455 nm > 96%; UV λ_{A}= 267 nm and 455 nm; MS: M-1 = 834.04 (calc 8.33.95).

δ-9H(1,3-dichloro-9,9-dimethylacridin-2-one-7=yl)-dideoxycytidine-5'-tetraphosphate (ddC4P-DDAO), δ-9H(1,3-dichloro-9,9-dimethylacridin-2-one-dideoxyadenosine-5'-tetraphosphate (ddA4P-DDAO) and δ-9H(1,3-dichloro-9,9-dimethylacridin-2-one-y-YL)-dideoxyguanosine-5'-tetraphosphate (ddG4P-DDAO) were synthesized and purified in a similar fashion. Analysis of these purified compounds provided the following data: ddC4P-DDAO: UV λ_{A} = 268 nm and 454 nm; MS: M-1 = 819.32 (calc 818.96); ddA4P-DDAO: UV λ_{A} = 263 nm and 457 nm; MS: M-1 = 843.30 (calc 842.97); ddG4P-DDAO: UV λ_{A}= 257 nm and 457 nm; MS: M-1 = 859.40 (calc 858.97).

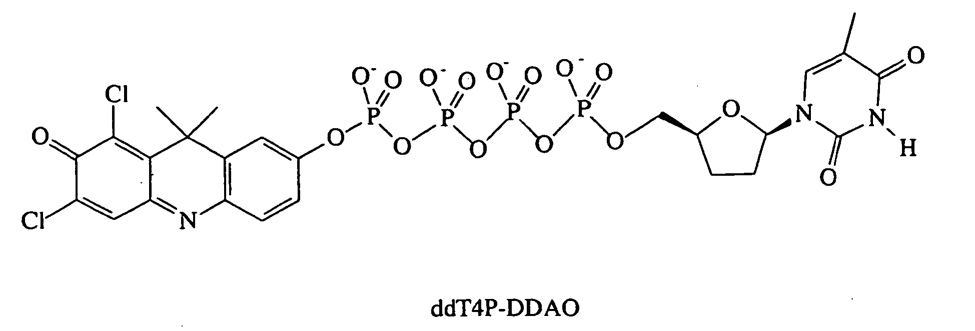

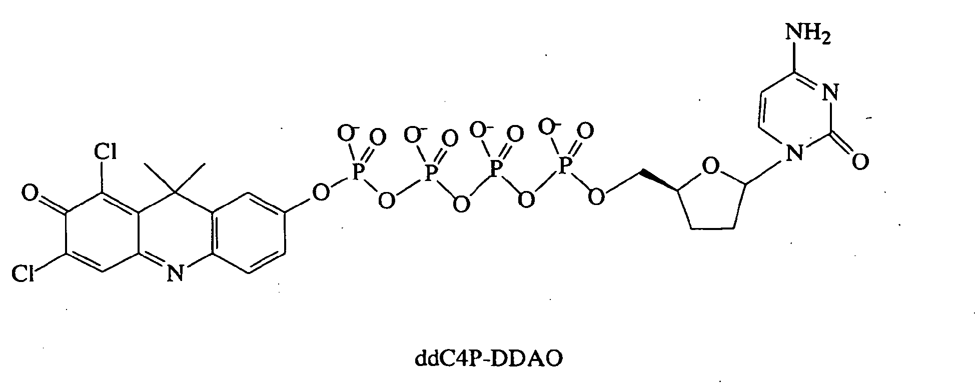

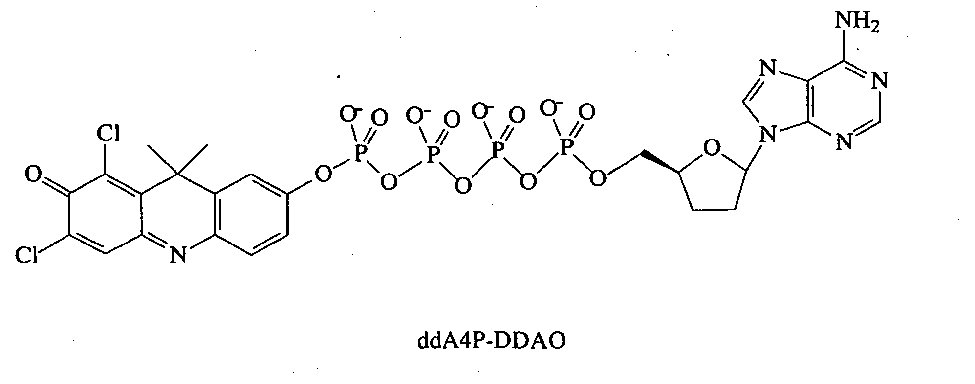

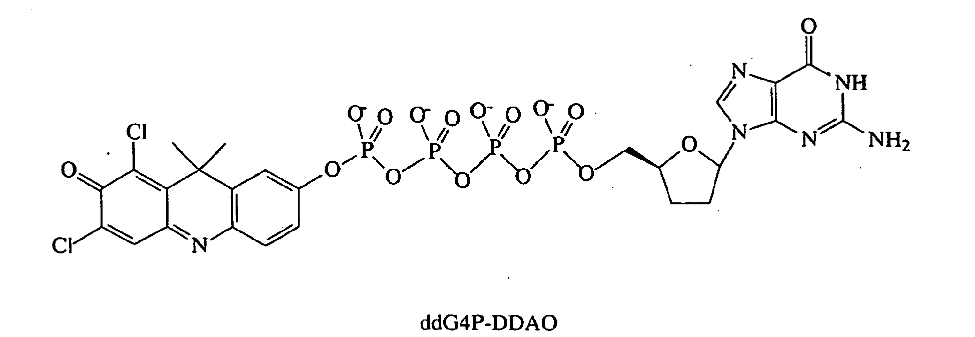

### Example 4

### Preparation of ε-9H (1,3-dichloro-9,9-dimethylacridin-2-one-7-yl)-dideoxythymidine-5'-pentaphosphate DDAO-ddT-pentaphosphate (ddT5P-DDAO)

### A. Preparation of DDAO pyrophosphate

DDAO-phosphate diammonium salt (11.8 µmol) was coevaporated with anhydrous DMF (3x 0.25 ml) and was dissolved in DMF (0.5 ml). To this carbonyldiimidazole (CDI; 9.6 mg, 5 eq) was added and the mixture was stirred at room temperature overnight. Excess CDI was destroyed by addition of MeOH (5 µl) and stirring for 30 minutes. To the mixture tributylammoniumdihydrogen phosphate (10 eq., 236 ml of 0.5 M solution in DMF) was added and the mixture was stirred at room temperature for 4 days. Reaction mixture was concentrated on rotavap. Residue was purified on HiPrep 16.10 Q XL column using 0-100% B using 0.1M TEAB/acetonitrle (3:1) as buffer A and 1 M TEAB/acetonitrile (3:1) as buffer B. Main peak (HPLC purity 98%) was collected, concentrated and coevaporated with methanol (2x). Residue was dissolved in 1 ml water to give 5.9 mM solution. UV/VIS λₘₐₓ = 456 nm.

### B. Preparation of ddT5P-DDAO

ddATP (100 µl of 47.5 mM solution in water) was coevaporated with anhydrous DMF (2x1 ml). To this DCC (5 eq., 4.9 mg) was added and mixture was coevaporated with DMF (1x1 ml). Residue was taken in anhydrous DMF (0.5 ml) and stirred at room temperature for 3 hours. To this 1.03 eq of DDAO pyrophosphate, separately coevaporated with anhydrous DMF (2x1 ml) was added as a DMF solution. Mixture was concentrated to dryness and then taken in 200 µl anhydrous DMF. Mixture was heated at 38°C for 2 days. Reaction mixture was concentrated, diluted with water, filtered and purified on HiTrap 5 ml ion exchange column using 0-100% A-B using a two step gradient. Solvent A = 0.1M TEAB/acetonitrile (3:1) and solvent B = 1M TEAB/acetonitrile (3:1). Fraction 12 and 13 which contained majority of product were combined, concentrated and coevaporated with methanol (2x). Residue was repurified on Xterra RP C-18 30-100 mm column using 0.30% acetonitrile in 0.1M TEAB in 5 column and 30-50% acetonitrile in 2 column volumes, flow rate 10 ml/min. Fraction containing pure product was concentrated and coevaporated with methanol (2x) and water (1x). HPLC purity at 455 nm> 99%. UVNIS = 268 nm and 455 nm. MS: M-1 = 914.03 (calc 913.93).

The DDAO dye attached to the gamma phosphate of these polyphosphates is fluorescent with an excitation maximum of 455 nm and an emission maximum of about 608 nm. Upon hydrolysis of the phosphate ester to release the free dye, the spectrum changes with excitation maximum of about 645 nm and emission maximum of about 659 nm. The change is readily detected by simple fluorescence measurements or color change.

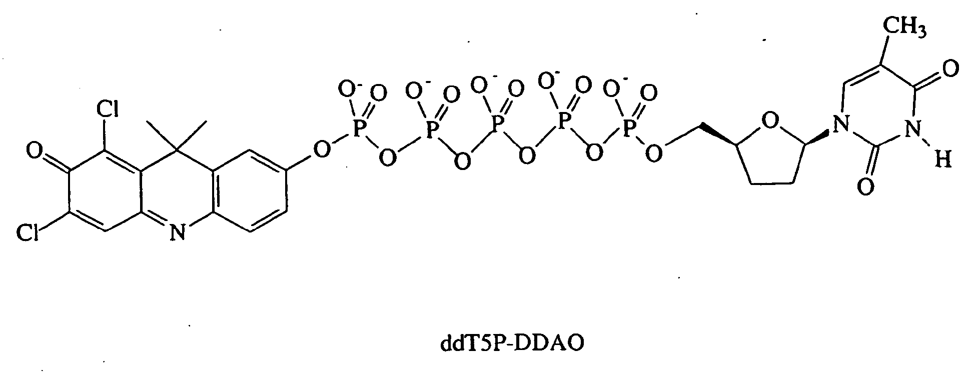

### Example 5

Preparation of δ-9H(1,3-dichloro-9,9-dimethylacridin-2-one-7-yl)- deoxythymidine-5'-tetraphosphate (dT4P-DDAO)

10 µmoles TTP TEA salt was evaporated to dryness. To the residue was added 40 µmoles tributylamine and 5 ml dry pyridine. The solution was reevaporated to dryness. After 2 coevaporations with 3ml dry dimethylformamide (DMF), residue was re-dissolved in 200 µl dry DMF, flushed with argon and stoppered. Using a syringe, 50 µmoles (8 mg) carbonyldiimidazole (CDI) dissolved in 100 µl dry DMF was added. The flask was stirred for 4 hr at ambient temperature.

While the above reaction was progressing, 35 mg (83 µmoles) DDAO phosphate and 166 µmoles tributylamine were dissolved in dry DMF. The DDAO phosphate was evaporated to dryness followed by 3 coevaporations with dry DMF. Residue was dissolved in 300 µl dry DMF.

After the 4 hr reaction time, 3.2 µl anhydrous methanol was added to the TTP-CDI reaction. The reaction was stirred 30 minutes. To this mixture, DDAO phosphate solution was added and mixture was stirred at ambient temperature for 18 hr. The reaction was checked by Reverse phase HPLC (Xterra 4.6x100 column, 0.1M TEAA/ acetonitrile). The reaction volume was reduced to 200 µl by evaporation and the reaction was allowed to progress for 80 hr.

After 80 hr, the reaction was stopped by adding 15 ml 0.1 M TEAB. The diluted mixture was applied to a 19x 100 Xterra RP column and eluted with an acetonitrile gradient in 0.1M TEAB. The fractions containing pure T4P-DDAO were evaporated to dryness and coevaporated twice with ethanol. The residue was reconstituted with MilliQ water. Yield: 1.10 µmoles, 11%; HPLC purity > 98% at 455 nm; MS: M-1 = 850.07 (calc. 849.95)

δ-9H(1,3-dichloro-9,9-dimethylacridin-2-one-7-yl)- deoxyguanosine-5'-tetraphosphate (dG4P-DDAO), δ-9H(1,3-dichloro-9,9-dimethylacridin-2-one-7-yl)-deoxycytidine-5'-tetraphosphate (dC4P-DDAO) and δ-9H(1,3-dichloro-9,9-dimethylacridin-2-one-7-yl)- deoxyadenosine-5'-tetraphosphate (dA4P-DDAO) were prepared in a similar manner as described above except 3.5 equivalents of DDAO phosphate was used instead of 8.3 equivalents. After C18 purification, samples were purified on ion exchange using a Mono Q 10/10 column.

δ-9H(1,3-dichloro-9,9-dimethylacridin-2-one-7-yl)- deoxyguanosine-5'-tetraphosphate (dG4P-DDAO): Yield 0.57 µmol, 5.7%; HPLC purity 99% at 455 nm; MS: M-1 = 875.03 (calc. 874.96).

δ-9H (1,3-dichloro-9,9-dimethylacridin-2-one-7-yl)- deoxycytidine-5'-tetraphosphate (dC4P-DDAO): Yield 0.24 µmole, 2.4%; HPLC purity 99% at 455 nm; MS: M-1 = 835.03 (calc. 834.95).

δ-9H (1,3-dichloro-9,9-dimethylacridin-2-one-7-yl)- deoxyadenosine-5'-tetraphosphate (dA4P-DDAO): Yield 0.38 µmole, 3.8%; HPLC purity 99% at 455 nm; MS: M-1 = 859.07 (calc. 858.97).

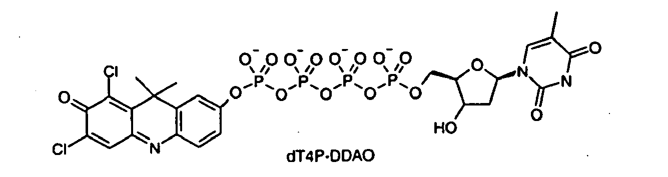

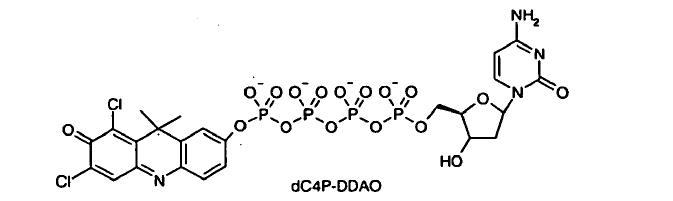

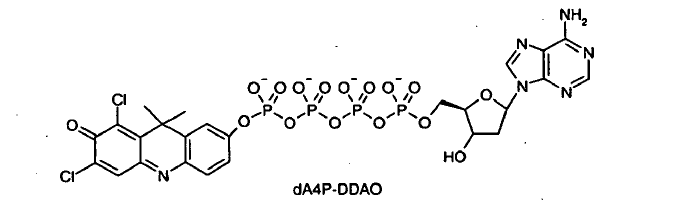

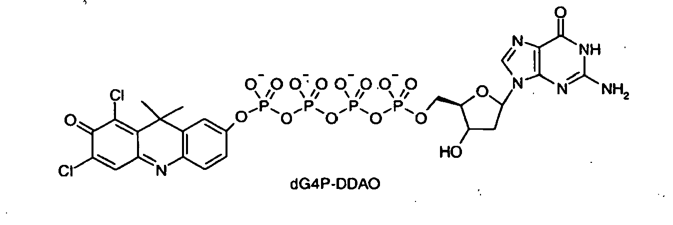

### Example 6

Use of exonuclease III to amplify signal generated by incorporation of nucleotides labeled on the terminal phosphate with fluorogenic dyes.

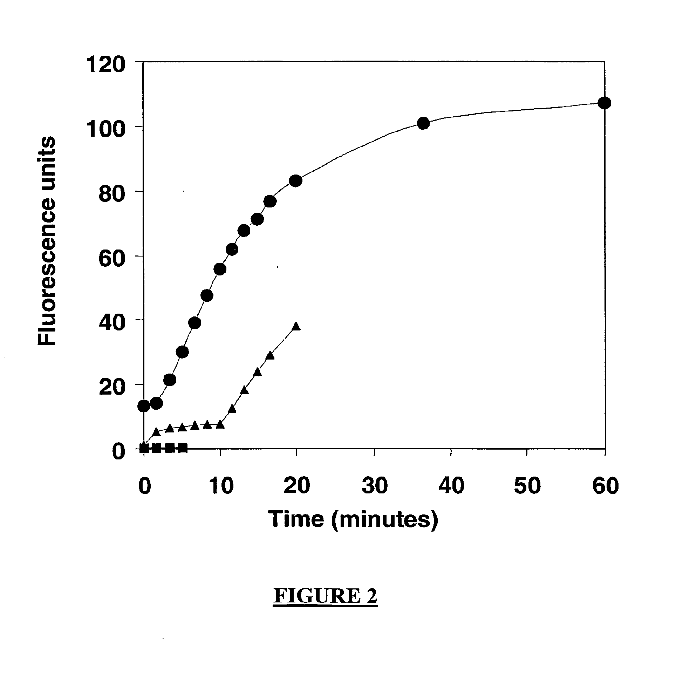

A 50 µl reaction containing 25 mM Tris HCl, pH 8.05 mM MgCl₂, 0.5 mM MnSO₄, 40 pmoles ddT4P-DDAO (DDAO-δ-2',3'-dideoxythymidine-5'-tetraphosphate), 5 pmoles primer (5'GTTTTCCCAGTCACGACGTTGT*A3' (SEQ ID NO: 1) where * is phosphorothioate linkage) and 10 pmoles template (5'GTCGTTATACAACGTCGTGACTGGGAAAA*ddC3' (SEQ ID NO: 2) where * is phosphorothioate linkage, ddC indicates a terminal dideoxynucleotide) was annealed by heating to 75° for 4 minutes and cooled to 21° C. Referring now to Figure 2, to this reaction was added the following: 0.15 units shrimp alkaline phosphatase and 0.5 units exonuclease III (squares) or 0.15 units shrimp alkaline phosphatase, 0.5 units exonuclease III, and 16 units Thermo Sequenase (circles). To a third reaction mixture, 0.15 units shrimp alkaline phosphatase and 16 units Thermo Sequenase were added, then after 10 minutes, 0.5 units exonuclease III was added (triangles). Reactions were incubated at room temperature in a quartz fluorescence ultra-microcuvet in an LS-55 Luminescence Spectrophotometer (Perkin Elmer), operated in time drive mode with excitation at 612 nm and emission at 670 nm. Emission is displayed in arbitrary units.

As shown in Figure 2, no fluorescence emission was obtained from the reaction mixture without polymerase. Moreover, as shown in Figure 2, amplification of the signal is only obtained when both exonuclease III and polymerase are present in the reaction mixture.

### Example 7

Use of exonuclease III to amplify signal generated by the sequence specific incorporation of nucleotides labeled on the terminal phosphate with fluorogenic dyes with sequence specificity.

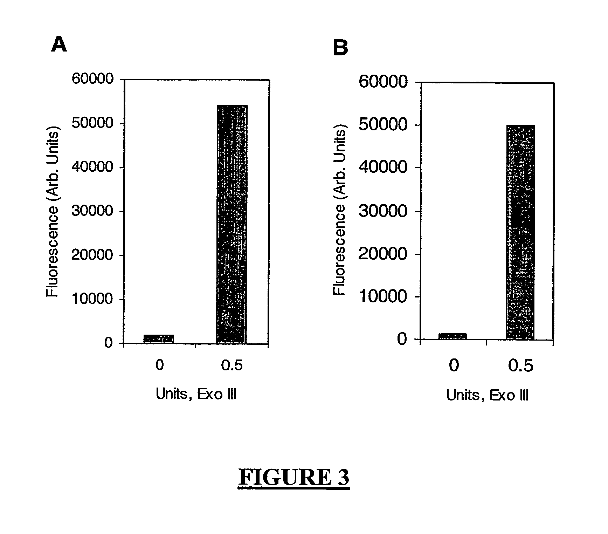

With reference to Figure 3A, an assay was performed to determine the presence of deoxycytidine (C) in the template. For each result shown in Figure 3A, a 50 µl reaction containing 25 mM Tris HCl, pH 8.0, 5 mM MgCl₂, 0.5 mM MnSO₄, 40 pmoles of ddG3P-resorufin (resorufin-γ-2',3'-dideoxyguanosine-5'-triphosphate), 5 pmoles primer (5'GTTTTCCCAGTCACGACGTTGT*A3' (SEQ ID NO: 1) where * is phosphorothioate linkage) and 10 pmoles template (5'GTCGTTCTACAACGTCGTGACTGGGAAAA*ddC3' (SEQ ID NO: 3) where * is phosphorothioate linkage, and ddC indicates a terminal dideoxynucleotide) was annealed by heating to 75° C for 4 minutes and cooled to 21° C. Thus, for Figure 3A the primer/template combination was:
5'GTTTTCCCAGTCACGACGTTGTA (SEQ ID NO: 1)
ddCAAAAGGGTCAGTGCTGCAACATCTTGCTG (SEQ ID NO: 3)

To this, 0.15 units shrimp alkaline phosphatase and 16 units Thermo Sequenase DNA polymerase were added, with exonuclease III added as indicated. The reaction was incubated at 21° C for 40 minutes. After incubation, 25 µl was removed to a 96 well plate and fluorescence was measured in a Tecan ULTRA plate reader with 530 nm excitation and 590 nm emission filters. Fluorescence emission is displayed in arbitrary units.

With reference now to Figure 3B, an assay was performed to determine the presence of deoxythymidine (T) in the template. For each result shown in Figure 3B, a 50 µl reaction containing 25 mM Tris HCl, pH 8.0, 5 mM MgCl₂, 0.5 mM MnSO₄, 40 pmoles of ddT4P-DDAO (DDAO-8-2',3'-dideoxythymidine-5'-tetraphosphate), 5 pmoles primer (5'GTTTTCCCAGTCACGACGTTGT*A3' (SEQ ID NO: 1) where * is phosphorothioate linkage) and 10 pmoles template (5'GTCGTTATACAACGTCGTGACTGGGAAAA*ddC3' (SEQ ID NO: 2) where * is phosphorothioate linkage, and ddC indicates a terminal dideoxynucleotide) was annealed by heating to 75° C for 4 minutes and cooled to 21° C. Thus, the primer/template combination was:
5' GTTTTCCCAGTCACGACGTTGTA (SEQ ID NO: 1)
ddCAAAAGGGTCAGTGCTGCAACATATTGCTG (SEQ ID NO: 2)

To this, 0.15 units shrimp alkaline phosphatase and 16 units Thermo Sequenase DNA polymerase were added, with exonuclease III added as indicated. The reaction was incubated at 21°C for 40 minutes. After incubation, 25 µl was removed to a 96 well plate and fluorescence was measured in a Tecan ULTRA plate reader with 612 nm excitation and 670 nm emission filters. Fluorescence emission is displayed in arbitrary units.

As shown in Figure 3A, for reactions containing the terminal-phosphate-labeled dideoxyguanosine triphosphate dye, fluorescence emission was detected for the Primer-Template combination where the next nucleotide in the template was a dC. With reference to Figure 3B, for reactions containing the terminal-phosphate-labeled dideoxythymidine tetraphosphate, fluorescence emission was detected for the Primer-Template combination where the next nucleotide in the template was a dA. Cleavage of the pyrophosphate product of phosphoryl transfer by shrimp alkaline phosphatase leads to a detectable change in the resorufin or DDAO label which allows for the detection of the nucleic acid, the synthesis and degradation of the complementary labeled nucleotide being repeated several times to effect the amplification of the signal.

### Example 8

### Detection of Streptavidin derivatized beads using a biotinylated oligo and ddA4P-methylcoumarin.

### A. Attachment of biotinylated oligo to streptavidin derivatized beads.

Streptavidin coated beads (100 µl, 10 mg/ml) were washed with 1x PBS-Tween (0.01%) 225 µl and 1x PBS, 225 µl. Beads were incubated with a mixture of 195 µl PBS-Tween (0.01%) and 5 µl of 50 µM solution of a biotinylated oligo (capable of forming a hairpin at 37oC, SEQ ID NO: 4 at 37oC for 30 min. After separation of supernatent, beads were washed with 0.5 ml PBS-Tween (0.01%), 0.5 ml of PBS buffer and resuspended in 0.5 ml PBS buffer. Oligo loading was determined to be 9 pmol/500µl of the final bead suspension, after cleavage of oligo from a portion of the beads with conc. NH4OH at 65oC for 10 min and measuring the fluorescence of fluorescein attached to the oligo. 
5'-(6-FAM)-ACG-TTT-TCT-TTA-TTG-TCA-GTC-GAC-CTA-GTC-GCT-CG
T
T*
3'-GsT-CAG-CTG-GAT-CAG-CGA-GA
T
SEQ ID NO: 4
 Where T* is a biotinylated thymidine base and s stands for phosphorothioate backbone that is resistant to Exo-III.

### B. Detection of streptavidin derivatized beads using ddA4P-methylcoumarin by repeated addition and removal of ddAMP to the biotinylated oligo.

50 µl of the oligo loaded beads (0.9 pmol oligo) were separated from the supernatent and washed with deionized water (50 µl) and reaction buffer (50 µl, 25 mM Hepes, pH 8.2, 5 mM MgCl2, 0.5 mM MnCl2, 0.01 % Tween-20, 0.0026 u/µl shrimp alkaline phosphatase, 1 mM DTT, 5 µM ddA4P-methylcoumarin, 0.065 u/µl Exo III and 0.012 u/µl TSI polymerase). Beads were incubated in the same reaction buffer at 37oC for 1.5h. Supernatent was separated and fluorescence was measured on Tecan ULTRA plate reader with excitation at 360 nm and emission at 465 nm. About 81% of starting ddA4P-methylcoumarin (202.6 pmol) was consumed giving an -225 fold amplification of signal.

### Example 9

### Detection of streptavidin derivatized beads using a biotinylated oligo and all four dN4P-methylcoumarin nucleotides (N = A,G,C & T)

50 µl of oligo-loaded beads per example 8A were separated from the supernatent and washed with deionized water (50 µl) and reaction buffer (50 µl, 25 mM Hepes, pH 8.2, 5 mM MgCl2, 0.5 mM MnCl2, 0.01% Tween-20, 0.0026 u/µl shrimp alkaline phosphatase, 1 mM DTT, 4.76 µM dA4P-methylcoumarin, 4.76 µM dG4P-methylcoumarin, 4.76 µM dC4P-methylcoumarin, 4.76 µM T4P-methylcoumarin, 0.065 u/µl Exo III and 0.012 u/µl TSI polymerase). Beads were incubated in the same reaction buffer at 37oC for 1.5h. Supernatent was separated and fluorescence was measured on Tecan ULTRA with excitation at 360 nm and emission at 465 nm. About 27% of starting dN4P-methylcoumarin nucleotides (257.7 pmol) were consumed giving an -286 fold amplification of signal.

### Example 10

Detection of streptavidin derivatized beads using a biotinylated oligo and three methylcoumarin labeled nucleotides (dA4P-methylcoumarin, dG4P-methylcoumarin, dC4P-methylcoumarin) and a DDAO labeled nucleotide (T4P-DDAO).

50 µl of oligo-loaded beads per example 8A were separated from the supernatent and washed with deionized water (50 µl) and reaction buffer (50 µl, 25 mM Hepes, pH 8.2, 5 mM MgCl2, 0.5 mM MnCl2,0.01% Tween-20, 0.0026 u/µl shrimp alkaline phosphatase, 1 mM DTT, 4.76 µM dA4P-methylcoumarin, 4.76 µM dG4P-methylcoumarin, 4.76 µM dC4P-methylcoumarin, 4.76 µM T4P-DDAO and 0.012 u/µl TSI polymerase). No Exo III was used. Beads were incubated in the same reaction buffer at 37oC for 1.5h. Supernatent was separated and fluorescence was measured on Tecan ULTRA with excitation at 360 nm and emission at 465 nm for methylcoumarin and excitation at 612 nm and emission at 670 nm for DDAO. Ratio of methylcoumarin and DDAO fluorescence counts was 7.1-7.4 very close to the expected value of 7.0 (ratio of the sum of A,G & C to T bases when the sequence is fully extended) indicating that sequence composition may be used to differentiate one analyte signal from another.

## Claims

1. A method of detecting an analyte comprising the steps of:
(a) anchoring said analyte to a nucleic acid template;
(b) conducting a nucleic acid polymerase reaction to produce labeled polyphosphate, said reaction comprising the reaction of said template, a primer, at least one terminal phosphate-labeled nucleotide, and a nucleic acid polymerase; and
(c) analyzing said labeled polyphosphate by fluorescent detection.

2. The method of claim 1, wherein said primer is a nuclease resistant primer.

3. The method of claim 2, wherein the nucleic acid polymerase reaction further includes an enzyme having 3' → 5' exonuclease activity.

4. The method of claim 1, wherein said analyzing step includes (a) reacting said labeled polyphosphate with a phosphatase to produce a detectable species characteristic of said analyte and (b) detecting said detectable species.

5. The method of claim 2, wherein said nuclease resistant primer includes a methyl phosphonate, a borano phosphate or a phosphorothioate linkage.

6. The method of claim 1, wherein the labels in at least one terminal phosphate-labeled nucleotide are enzyme-activatable labels selected from the group consisting of chemiluminescent compounds, fluorogenic dyes, chromogenic dyes, mass tags, electrochemical tags and combinations thereof.

7. The method of claim 1, wherein at least one terminal-phosphate-labeled nucleotide is represented by the formula: 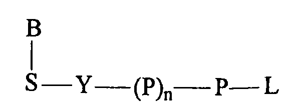 wherein P is phosphate (PO₃) and derivatives thereof, n is 2 or greater; Y is an oxygen or sulfur atom; B is a nitrogen-containing heterocyclic base; S is an acyclic moiety, carbocyclic moiety or sugar moiety; L is an enzyme-activatable label containing a hydroxyl group, a sulfhydryl group or an amino group suitable for forming a phosphate ester, a thioester or a phosphoramidate linkage at the terminal phosphate of a natural or modified nucleotide; and P-L is a phosphorylated label which preferably becomes independently detectable when the phosphate is removed.

8. A method of detecting an analyte comprising the steps of:
(a) anchoring said analyte to;
(i) a primer, or
(ii) a hairpin structure which comprises a nucleic acid template and primer,
(b) conducting a nucleic acid polymerase reaction to produce labeled polyphosphate, said reaction comprising the reaction of said template, a primer, at least one terminal phosphate-labeled nucleotide, and a nucleic acid polymerase; and
(c) analyzing said labeled polyphosphate by fluorescent detection.

9. A method of detecting and characterizing multiple analytes in a sample, comprising the steps of:
(a) anchoring to each analyte a specific template nucleic acid sequence with a unique base at the site opposite to the complementary nucleotide being added;
(b) conducting a DNA polymerase reaction to produce labeled polyphosphates, said reaction comprising the reaction of said templates, primers complementary to said specific template sequence, two or more terminal phosphate-labeled nucleotides with different labels, a DNA polymerase and an enzyme having 3' → 5' exonuclease activity;
(c) permitting said labeled polyphosphates to react with a phosphatase to produce detectable species unique to each of said analytes; and
(d) detecting said detectable species.

10. A method of detecting and characterizing multiple analytes in a sample, comprising the steps of:
(a) anchoring to each analyte a specific template nucleic acid sequence with a unique base at the site opposite to the complementary nucleotide being added;
(b) conducting a DNA polymerase reaction to produce uniquely labeled polyphosphates; said reaction comprising the reaction of said templates, nuclease resistant primers complementary to said specific target sequence of each of said multiple analytes, two or more terminal phosphate-labeled nucleotides having 4 or more phosphate groups in the polyphosphate chain and each bearing a different label, a DNA polymerase and an enzyme having 3' → 5' exonuclease activity; and
(c) detecting the labeled polyphosphates.

11. A method of detecting and characterizing multiple analytes in a reaction compartment, comprising the steps of:
(a) anchoring a unique template nucleic acid sequence to each of said analytes;
(b) anchoring said analytes to the surface of said reaction compartment;
(c) conducting a DNA polymerase reaction to produce labeled polyphosphate; said reaction comprising the reaction of the unique template sequence of one of said analytes, a nuclease resistant primer complementary to said unique template sequence, at least one terminal phosphate-labeled nucleotides having 4 or more phosphate groups in the polyphosphate chain, a DNA polymerase and an enzyme having 3' → 5' exonuclease activity;
(d) detecting said labeled polyphosphate;
(e) washing off the unanchored components; and
(f) repeating steps (a) to (d) with a nuclease resistant primer complementary to another unique template sequence of a different analyte until all the analytes are analyzed.

12. The method of claim 11, wherein said at least one terminal phosphate labeled nucleotides have 4 or more phosphate groups in the polyphosphate chain.

13. A kit for detecting an analyte suitable for the method of any of claims 1-12 comprising;
(a) at least one terminal-phosphate-labeled nucleotide;
(b) DNA polymerase; and
(c) phosphatase.
(d) at least one nucleic acid template; and
(e) at least one nuclease resistant primer complementary to said at least one nucleic acid template;
wherein said at least one nucleic acid template and/or said complementary nuclease resistant primer has an anchoring moiety.

14. A kit for detecting an analyte according to claim 13 further comprising at least one hairpin template-primer combination with a nuclease resistant 3'-end.

## Patentansprüche

1. Verfahren zum Nachweisen eines Analyten, umfassend die Schritte:
(a) Verankern des Analyten an einem Nukleinsäure-Template;
(b) Durchführen einer Nukleinsäurepolymerasereaktion, um markiertes Polyphosphat herzustellen, die Reaktion umfassend die Reaktion des Templates, eines Primers, zumindest eines am terminalen Phosphat markierten Nukleotids und einer Nukleinsäurepolymerase; und
(c) Analysieren des markierten Polyphosphats durch Fluoreszenzdetektion.

2. Verfahren nach Anspruch 1, wobei der Primer ein nukleaseresistenter Primer ist.

3. Verfahren nach Anspruch 2, wobei die Nukleinsäurepolymerasereaktion weiterhin ein Enzym mit 3' → 5' Exonukleaseaktivität beinhaltet.

4. Verfahren nach Anspruch 1, wobei der Analyseschritt (a) das Umsetzen des markierten Polyphosphats mit einer Phosphatase beinhaltet, um eine nachweisbare Spezies herzustellen, die charakteristisch für den Analyten ist, und (b) das Nachweisen der nachweisbaren Spezies.

5. Verfahren nach Anspruch 2, wobei der nukleaseresistente Primer eine Methylphosphonat-, eine Boranophosphat- oder eine Phosphorthioat-Bindung beinhaltet.

6. Verfahren nach Anspruch 1, wobei die Markierungen bei zumindest einem am terminalen Phosphat markierten Nukleotid Enzym-aktivierbare Markierungen sind, die aus jener Gruppe ausgewählt sind, die besteht aus chemilumineszenten Verbindungen, fluorogenen Farbstoffen, chromogenen Farbstoffen, Mass-Tags, elektrochemischen Tags und Kombinationen davon.

7. Verfahren nach Anspruch 1, wobei zumindest ein am terminalen Phosphat markiertes Nukleotid dargestellt wird durch die Formel: 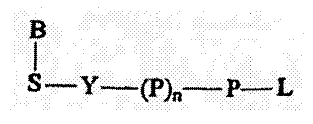 wobei P für Phosphat (PO₃) und Derivate davon steht, n für 2 oder mehr steht; Y für ein Sauerstoff- oder Schwefelatom steht; B für eine stickstoffhaltige heterocyclische Base steht; S für einen acyclischen Teil, carbocyclischen Teil oder Zuckerteil steht; L für eine Enzym-aktivierbare Markierung steht, die eine Hydroxygruppe, eine Sulfhydrylgruppe oder eine Aminogruppe enthält, geeignet zur Bildung einer Phosphatester-, einer Thioester- oder einer Phosphoramidat-Bindung am terminalen Phosphat eines natürlichen oder modifizierten Nukleotids; und P-L für eine phosphorylierte Markierung steht, die vorzugsweise unabhängig nachweisbar wird, wenn das Phosphat entfernt wird.

8. Verfahren zum Nachweisen eines Analyten, umfassend die Schritte:
(a) Verankern des Analyten an;
(i) einem Primer, oder
(ii) einer Haarnadelstruktur, die ein Nukleinsäure-Template und einen Primer umfasst,
(b) Durchführen einer Nukleinsäurepolymerasereaktion, um markiertes Polyphosphat herzustellen, die Reaktion umfassend die Reaktion des Templates, eines Primers, zumindest eines am terminalen Phosphat markierten Nukleotids und einer Nukleinsäurepolymerase; und
(c) Analysieren des markierten Polyphosphats durch Fluoreszenzdetektion.

9. Verfahren zum Nachweisen und Charakterisieren mehrerer Analyten in einer Probe, umfassend die Schritte:
(a) Verankern, an jedem Analyten, einer spezifischen Template-Nukleinsäuresequenz mit einer unikalen Base an der Stelle gegenüber dem komplementären Nukleotid, das hinzugefügt wird;
(b) Durchführen einer DNA-Polymerasereaktion, um markierte Polyphosphate herzustellen, die Reaktion umfassend die Reaktion der Templates, zur spezifischen Template-Sequenz komplementärer Primer, zweier oder mehr am terminalen Phosphat markierter Nukleotide mit unterschiedlichen Markierungen, einer DNA-Polymerase und eines Enzyms mit 3' → 5' Exonukleaseaktivität;
(c) Ermöglichen, dass die markierten Polyphosphate mit einer Phosphatase reagieren, um nachweisbare Spezies herzustellen, die für jeden der Analyten eindeutig sind; und
(d) Nachweisen der nachweisbaren Spezies.

10. Verfahren zum Nachweisen und Charakterisieren mehrerer Analyten in einer Probe, umfassend die Schritte:
(a) Verankern, an jedem Analyten, einer spezifischen Template-Nukleinsäuresequenz mit einer unikalen Base an der Stelle gegenüber dem komplementären Nukleotid, das hinzugefügt wird;
(b) Durchführen einer DNA-Polymerasereaktion, um eindeutig markierte Polyphosphate herzustellen; die Reaktion umfassend die Reaktion der Templates, nukleaseresistenter Primer, die komplementär zur spezifischen Target-Sequenz jedes der mehreren Analyten sind, zweier oder mehr am terminalen Phosphat markierter Nukleotide, die 4 oder mehr Phosphatgruppen in der Polyphosphatkette aufweisen und jeweils eine andere Markierung tragen, einer DNA-Polymerase und eines Enzyms mit 3' → 5' Exonukleaseaktivität; und
(c) Nachweisen der markierten Polyphosphate.

11. Verfahren zum Nachweisen und Charakterisieren mehrerer Analyten in einem Reaktionskompartiment, umfassend die Schritte:
(a) Verankern einer unikalen Template-Nukleinsäuresequenz an jedem der Analyten;
(b) Verankern der Analyten an der Oberfläche des Reaktionskompartiments;
(c) Durchführen einer DNA-Polymerasereaktion, um markiertes Polyphosphat herzustellen; die Reaktion umfassend die Reaktion der unikalen Template-Sequenz eines der Analyten, eines nukleaseresistenten Primers, der zur unikalen Template-Sequenz komplementär ist, zumindest eines am terminalen Phosphat markierten Nukleotids mit 4 oder mehr Phosphatgruppen in der Polyphosphatkette, einer DNA-Polymerase und eines Enzyms mit 3' → 5' Exonukleaseaktivität;
(d) Nachweisen des markierten Polyphosphats;
(e) Abwaschen der unverankerten Komponenten; und
(f) Wiederholen der Schritte (a) bis (d) mit einem nukleaseresistenten Primer, der zu einer anderen unikalen Template-Sequenz eines anderen Analyten komplementär ist, bis alle der Analyten analysiert sind.

12. Verfahren nach Anspruch 11, wobei das zumindest eine am terminalen Phosphat markierte Nukleotid 4 oder mehr Phosphatgruppen in der Polyphosphatkette aufweist.

13. Für das Verfahren nach einem der Ansprüche 1 - 12 geeignetes Kit zum Nachweisen eines Analyten, umfassend;
(a) zumindest ein am terminalen Phosphat markiertes Nukleotid;
(b) DNA-Polymerase; und
(c) Phosphatase;
(d) zumindest ein Nukleinsäure-Template; und
(e) zumindest einen nukleaseresistenten Primer, der komplementär zum zumindest einen Nukleinsäure-Template ist;
wobei das zumindest eine Nukleinsäure-Template und/oder der komplementäre nukleaseresistente Primer einen Verankerungsteil aufweist.

14. Kit zum Nachweisen eines Analyten nach Anspruch 13, das weiterhin zumindest eine Haarnadel-Template/Primer-Kombination mit einem nukleaseresistenten 3'-Ende umfasst.

## Revendications

1. Procédé de détection d'un analyte comprenant les étapes de :
(a) ancrage dudit analyte sur une matrice d'acide nucléique ;
(b) mise en oeuvre d'une réaction de polymérase d'acide nucléique afin de produire un polyphosphate marqué, ladite réaction comprenant la réaction de ladite matrice, d'une amorce, d'au moins un nucléotide à phosphate terminal marqué, et d'une polymérase d'acide nucléique ; et
(c) l'analyse dudit polyphosphate marqué par détection de fluorescence.

2. Procédé selon la revendication 1, dans lequel ladite amorce est une amorce résistant aux nucléases.

3. Procédé selon la revendication 2, dans lequel la réaction de polymérase d'acide nucléique comporte, en outre, une enzyme présentant une activité exonucléase 3' → 5'.

4. Procédé selon la revendication 1, dans lequel ladite étape d'analyse comporte
(a) la réaction dudit polyphosphate marqué avec une phosphatase afin de produire une espèce détectable caractéristique dudit analyte et (b) la détection de ladite espèce détectable.

5. Procédé selon la revendication 2, dans lequel ladite amorce résistant à la nucléase comporte une liaison méthyle phosphonate, borano phosphate ou phosphorothioate.

6. Procédé selon revendication 1, dans lequel les marqueurs dans au moins un nucléotide à phosphate terminal marqué sont des marqueurs pouvant être activés par enzyme sélectionnés à partir du groupe constitué par des composés chimioluminescents, des colorants fluorogènes, des colorants chromogènes, des étiquettes massiques, des étiquettes électrochimiques et des mélanges de ceux-ci.

7. Procédé selon la revendication 1, dans lequel au moins un nucléotide à phosphate terminal marqué est représenté par la formule : 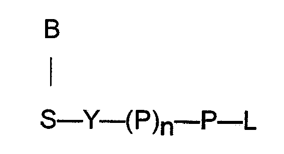 dans laquelle P est du phosphate (PO3) et des dérivés de celui-ci, n est supérieur ou égal à 2 ; Y est un atome d'oxygène ou de soufre B est une base hétérocyclique contenant de l'azote ; S est une fraction acyclique, une fraction carbocyclique ou une fraction sucre ; L est une étiquette pouvant être activée par enzyme contenant un groupe hydroxyle, un groupe sulfhydryle ou un groupe amino approprié pour la formation d'une liaison phosphate ester, thioester ou phosphoramidate au niveau du phosphate terminal d'un nucléotide naturel ou modifié ; et P-L est une étiquette phosphorylée qui, de préférence, devient détectable de manière indépendante lorsque le phosphate est éliminé.

8. Procédé de détection d'un analyte comprenant les étapes de :
(a) ancrage dudit analyte sur :
(i) une amorce, ou
(ii) une structure en épingle à cheveux qui comprend une matrice d'acide nucléique et une amorce,
(b) mise en oeuvre d'une réaction de polymérase d'acide nucléique afin de produire un polyphosphate marqué, ladite réaction comprenant une réaction de ladite matrice, d'une amorce, d'au moins un nucléotide à phosphate terminal marqué, et d'une polymérase d'acide nucléique ; et
(c) analyse dudit polyphosphate marqué par détection de fluorescence.

9. Procédé de détection et de caractérisation d'analytes multiples dans un échantillon, comprenant les étapes de :
(a) ancrage, sur chaque analyte, d'une séquence d'acide nucléique de matrice spécifique avec une base unique au niveau du site opposé au nucléotide complémentaire qui est ajouté ;
(b) mise en oeuvre d'une réaction d'ADN polymérase afin de produire des polyphosphates marqués, ladite réaction comprenant la réaction desdites matrices, d'amorces complémentaires à ladite séquence de matrice spécifique, de deux ou plusieurs nucléotides à phosphate terminal marqué avec différentes étiquettes, d'une ADN polymérase et d'une enzyme présentant une activité exonucléase 3' → 5' ;
(c) autorisation de la réaction desdits polyphosphates marqués avec une phosphatase afin de produire des espèces détectables uniques à chacun desdits analytes ; et
(d) détection desdites espèces détectables.

10. Procédé de détection et de caractérisation d'analytes multiples dans un échantillon, comprenant les étapes de :
(a) ancrage sur chaque analyte d'une séquence d'acide nucléique de matrice spécifique avec une base unique au niveau du site opposé au nucléotide complémentaire qui est ajouté ;
(b) mise en oeuvre d'une réaction d'ADN polymérase afin de produire des polyphosphates marqués de manière unique ; ladite réaction comprenant la réaction desdites matrices, d'amorces résistantes à la nucléase complémentaires de ladite séquence cible spécifique de chacun desdits analytes multiples, de deux ou plusieurs nucléotides à phosphate terminal marqué comportant 4 groupes phosphates ou plus dans la chaîne polyphosphate et chacun portant une étiquette différente, d'une ADN polymérase et d'une enzyme présentant une activité exonucléase 3' → 5' ; et
(c) détection des polyphosphates marqués.

11. Procédé de détection et de caractérisation d'analytes multiples dans un compartiment de réaction, comprenant les étapes de :
(a) ancrage d'une séquence d'acide nucléique à matrice unique sur chacun desdits analytes ;
(b) ancrage desdits analytes sur la surface dudit compartiment de réaction ;
(c) mise en oeuvre d'une réaction d'ADN polymérase afin de produire un polyphosphate marqué ; ladite réaction comprenant la réaction de la séquence de matrice unique de l'un desdits analytes, d'une amorce résistant à la nucléase complémentaire de ladite séquence de matrice unique, d'au moins un nucléotide à phosphate terminal marqué comportant 4 groupe phosphates ou plus dans la chaîne polyphosphate, d'une ADN polymérase et d'une enzyme présentant une activité exonucléase 3' → 5' ;
(d) détection dudit polyphosphate marqué ;
(e) lavage des composants non ancrés ; et
(f) répétition des étapes (a) à (d) avec l'amorce résistant à la nucléase complémentaire d'une autre séquence de matrice unique d'un analyte différent jusqu'à ce que tous les analytes ont été analysés.

12. Procédé selon la revendication 11, dans lequel lesdits au moins un nucléotides à phosphate terminal marqué comportent 4 groupes phosphates ou plus dans la chaîne polyphosphate.

13. Kit de détection d'un analyte approprié pour le procédé selon l'une quelconque des revendications 1 à 12 comprenant ;
(a) au moins un nucléotide à phosphate terminal marqué ;
(b) une ADN polymérase ; et
(c) de la phosphatase.
(d) au moins une matrice d'acide nucléique ; et
(e) au moins une amorce résistant à la nucléase complémentaire de ladite au moins une matrice d'acide nucléique ;
dans lequel ladite au moins une matrice d'acide nucléique et/ou ladite amorce résistant à la nucléase complémentaire présente une fraction d'ancrage.

14. Kit de détection d'un analyte selon la revendication 13, comprenant, en outre, au moins une association de matrice en épingle à cheveux - amorce avec une extrémité 3' résistant à la nucléase.


### SEQUENCE LISTING

<110> Sood, Anup
   Kumar, Shiv
   Fuller, Carl
   Nelson, John
<120> Analyte Detection
<130> PB0271
<140> PCT/US2003/27285
   <141> 2003-08-29
<150> US 60/406,893
   <151> 2002-08-29
<160> 4
<170> PatentIn version 3.2
<210> 1
   <211> 23
   <212> DNA
   <213> artificial sequence
<220>
   <223> synthetic oligonucleotide
<400> 1
   gttttcccag tcacgacgtt gta 23
<210> 2
   <211> 30
   <212> DNA
   <213> artificial sequence
<220>
   <223> synthetic oligonucleotide
<400> 2
   gtcgttatac aacgtcgtga ctgggaaaac 30
<210> 3
   <211> 30
   <212> DNA
   <213> artificial sequence
<220>
   <223> synthetic oligonucleotide
<400> 3
   gtcgttctac aacgtcgtga ctgggaaaac 30
<210> 4
   <211> 57
   <212> DNA
   <213> artificial sequence
<220>
   <223> synthetic oligonucleotide
<400> 4
   acgttttctt tattgtcagt cgacctagtc gctcgtttag agcgactagg tcgactg 57